(19) Europäisches Patentamt
European Patent Office
Office européen des brevets

(11) EP 3 620 536 A1

(12) EUROPEAN PATENT APPLICATION

(43) Date of publication:
11.03.2020 Bulletin 2020/11

(51) Int Cl.:
*C12Q 1/6876* (2018.01) *C12Q 1/6879* (2018.01)

(21) Application number: 18382646.0

(22) Date of filing: 07.09.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Consejo Superior De Investigaciones Científicas
28006 Madrid (ES)
- Institut Français de Recherche pour l'Exploitation de la Mer (IFREMER)
92138 Issy-les-Moulineaux Cedex (FR)

(72) Inventors:
- PIFERRER CIRCUNS, Francesc
08003 Barcelona (ES)
- ANASTASIADI, Dafni
08003 Barcelona (ES)
- SÁNCHEZ BAIZÁN, Núria Sánchez Baizán
08003 Barcelona (ES)
- ALLAL, François
92138 Issy-Les-Moulineaux (FR)
- VANDEPUTTE, Marc
34250 Palavas les Flots (FR)

(74) Representative: Pons
Glorieta Ruben Dario 4
28010 Madrid (ES)

# (54) METHOD FOR PREDICTING SEX IN FISH

(57) The present invention relates to an *in vitro* method for predicting the sex in fish comprising (a) Determining the methylation levels at the CpG sites of (i) the *dmrt1* gene promoter within the DNA region comprising the sequence SEQ ID NO: 1, and (ii) the *cyp19a1a* gene promoter within the DNA region comprising the sequence SEQ ID NO: 2 , and (b) determining the probability of being a female, $F^P$, wherein if $F^P > 0.5$ the fish is a female and if $F^P < 0.5$ the fish is a male.

EP 3 620 536 A1

SEQUENCE LISTING

<110> CONSEJO SUPERIOR DE INVESTIGACIONES CIENTÍFICAS (CSIC)
INSTITUT FRANÇAIS DE RECHERCHE POUR L'EXPLOITATION DE LA MER (IFREMER)

<120> Method for predicting sex in fish

<130> EP1641.1412

<160> 19

<170> PatentIn version 3.5

<210> 1
<211> 445
<212> DNA
<213> Dicentrarchus labrax

<220>
<221> GC_signal
<222> (46)..(46)
<223> Position 18,505,626 from the chromosome 20 of the European seabass genome (dicLab1), version 1.0c as annotated by the official public site of access to the genome (http://seabass.mpipz.de/) and accessed in August 2018.

<220>
<221> GC_signal
<222> (57)..(57)
<223> Position 18,505,637 from the chromosome 20 of the European seabass genome (dicLab1), version 1.0c as annotated by the official public site of access to the genome (http://seabass.mpipz.de/) and accessed in August 2018.

<220>
<221> GC_signal
<222> (74)..(74)
<223> Position 18,505,680 from the chromosome 20 of the European seabass genome (dicLab1), version 1.0c as annotated by the official public site of access to the genome (http://seabass.mpipz.de/) and accessed in August 2018.

<220>
<221> GC_signal
<222> (108)..(108)
<223> Position 18,505,688 from the chromosome 20 of the European seabass genome (dicLab1), version 1.0c as annotated by the official public site of access to the genome (http://seabass.mpipz.de/) and accessed in August 2018.

<220>
<221> GC_signal
<222> (118)..(118)
<223> Position 18,505,698 from the chromosome 20 of the European seabass genome (dicLab1), version 1.0c as annotated by the official public site of access to the genome (http://seabass.mpipz.de/) and accessed in August 2018.

<220>
<221> GC_signal
<222> (152)..(152)

<223> Position 18,505,732 from the chromosome 20 of the European seabass genome (dicLab1), version 1.0c as annotated by the official public site of access to the genome (http://seabass.mpipz.de/) and accessed in August 2018.

<220>
<221> GC_signal
<222> (167)..(167)
<223> Position 18,505,747 from the chromosome 20 of the European seabass genome (dicLab1), version 1.0c as annotated by the official public site of access to the genome (http://seabass.mpipz.de/) and accessed in August 2018.

<220>
<221> GC_signal
<222> (211)..(211)
<223> Position 18,505,791 from the chromosome 20 of the European seabass genome (dicLab1), version 1.0c as annotated by the official public site of access to the genome (http://seabass.mpipz.de/) and accessed in August 2018.

<220>
<221> GC_signal
<222> (217)..(217)
<223> Position 18,505,797 from the chromosome 20 of the European seabass genome (dicLab1), version 1.0c as annotated by the official public site of access to the genome (http://seabass.mpipz.de/) and accessed in August 2018.

<220>
<221> GC_signal
<222> (310)..(310)
<223> Position 18,505,890 from the chromosome 20 of the European seabass genome (dicLab1), version 1.0c as annotated by the official public site of access to the genome (http://seabass.mpipz.de/) and accessed in August 2018.

<220>
<221> GC_signal
<222> (324)..(324)
<223> Position 18,505,904 from the chromosome 20 of the European seabass genome (dicLab1), version 1.0c as annotated by the official public site of access to the genome (http://seabass.mpipz.de/) and accessed in August 2018.

<220>
<221> GC_signal
<222> (365)..(365)
<223> Position 18,505,945 from the chromosome 20 of the European seabass genome (dicLab1), version 1.0c as annotated by the official public site of access to the genome (http://seabass.mpipz.de/) and accessed in August 2018.

<220>
<221> GC_signal
<222> (379)..(379)
<223> Position 18,505,959 from the chromosome 20 of the European seabass genome (dicLab1), version 1.0c as annotated by the official public site of access to the genome (http://seabass.mpipz.de/) and accessed in August 2018.

<220>
<221> GC_signal

<222> (410)..(410)
<223> Position 18,505,990 from the chromosome 20 of the European seabass genome (dicLab1), version 1.0c as annotated by the official public site of access to the genome (http://seabass.mpipz.de/) and accessed in August 2018.

<400> 1
taactgagag tgggtgcatg ccactgctct gccagttctc tcccccgctt ccagctcgtt 60

tcctcccaca agtcgcagca gcagcagcag cagcagcagc ggcagggcgg ggcaggacgg 120

tgaccttata gcctccagcc tggcaccact gcgagcttta accagccgag cctgctggga 180

caaactctaa catagtagct aaagtctgac cgctgtcgtc taccagttag tgcaccagac 240

ttgtgttgca gttttatttc cagcagccat gagcaaggac aagcagagca agcaggtgcc 300

agagtccacc ggacctctgt ccccgtccaa aggccagaaa ccccccagga tgcccaagtg 360

ttcccgctgt aggaaccacg gctatgtgtc tcctctgaag ggacacaagc gcttttgcaa 420

ctggagggac tgtcagtgtc ccaaa 445

<210> 2
<211> 523
<212> DNA
<213> Dicentrarchus labrax

<220>
<221> GC_signal
<222> (15)..(15)
<223> Position 21,022,651 from chromosome 5 of the European seabass genome (dicLab1), version 1.0c as annotated by the official public site of access to the genome (http://seabass.mpipz.de/) and accessed in August 2018.

<220>
<221> GC_signal
<222> (388)..(388)
<223> Position 21,022,278 from chromosome 5 of the European seabass genome (dicLab1), version 1.0c as annotated by the official public site of access to the genome (http://seabass.mpipz.de/) and accessed in August 2018.

<220>
<221> GC_signal
<222> (395)..(395)
<223> Position 21,022,271 from chromosome 5 of the European seabass genome (dicLab1), version 1.0c as annotated by the official public site of access to the genome (http://seabass.mpipz.de/) and accessed in August 2018.

<220>
<221> GC_signal
<222> (411)..(411)
<223> Position 21,022,255 from chromosome 5 of the European seabass genome (dicLab1), version 1.0c as annotated by the official public site of access to the genome (http://seabass.mpipz.de/) and accessed in August 2018.

<220>

<221> GC_signal
<222> (431)..(431)
<223> Position 21,022,235 from chromosome 5 of the European seabass genome (dicLab1), version 1.0c as annotated by the official public site of access to the genome (http://seabass.mpipz.de/) and accessed in August 2018.

<220>
<221> GC_signal
<222> (452)..(452)
<223> Position 21,022,214 from chromosome 5 of the European seabass genome (dicLab1), version 1.0c as annotated by the official public site of access to the genome (http://seabass.mpipz.de/) and accessed in August 2018.

<400> 2
tattccagct ttccgttgtc tgtcttcctt tatagataca aaagtaaata aaacttgtgt 60

ggatttttct agaaaaaaac ttcaattcaa ttttgcaaga cagataaagc tttaacaata 120

ataataacaa taacagactt tcccatattc tcagaggcca gatgttcttt tgtagggttt 180

ccaatcagat tattttgatg ttcaaaggag gagcaaaact tgcttttcat cactaaaatg 240

tcaaataacc ccactccaac tccaacatgt actcactgaa ctaagtcctg tactctcaag 300

agcacaggca ctaatacaac ccttcaaggt tacaagtgta ttgtttacca tttttctcct 360

ctgttgtggc ttttactttg ccctgacgtg gctcgtaacc agctcagacc gcatataaag 420

agaaagcccc gattgttgag gcagctcaca cggagcagtt gcttttggtt attttaaatg 480

gatctgatct ctgcatgtga acgggcaatg actcctgtag gtt 523

<210> 3
<211> 301
<212> DNA
<213> Dicentrarchus labrax

<220>
<221> GC_signal
<222> (94)..(94)
<223> Position 20,275,623 from chromosome 1A of the European seabass genome (dicLab1), version 1.0c as annotated by the official public site of access to the genome (http://seabass.mpipz.de/) and accessed in August 2018.

<220>
<221> GC_signal
<222> (208)..(208)
<223> Position 20,275,509 from chromosome 1A of the European seabass genome (dicLab1), version 1.0c as annotated by the official public site of access to the genome (http://seabass.mpipz.de/) and accessed in August 2018.

<220>
<221> GC_signal
<222> (293)..(293)
<223> Position 20,275,424 from chromosome 1A of the European seabass genome (dicLab1), version 1.0c as annotated by the official public site of access to the genome (http://seabass.mpipz.de/) and accessed in August 2018.

<400> 3
gtgggaaact cctctcacat tcccaggaat gacaaatatt cactccctac ttggctggtc 60

tggactcaga gtgaccatgt tcctgcaaca gtggcgactg attttggctt tgggtgagtg 120

catttgctct taaaaaaacc cctctgtttc ttgtgtttct ttctattttg ttttacattt 180

caatcttaaa atgaaagact tagatatgcg tgttttgctt ggatagtgac agtagagaga 240

cacaggaaag tcaggggaga gagaggggat gacttgcagc aaagggtcca ggccgctgtg 300

t 301

<210> 4
<211> 23
<212> DNA
<213> Artificial Sequence

<220>
<223> Forward primer used for amplifying the cyp19a1a gene

<400> 4
ttagtttttc gttgtttgtt ttt 23

<210> 5
<211> 25
<212> DNA
<213> Artificial Sequence

<220>
<223> Reverse Primer used for amplifying the cyp19a1a gene

<400> 5
acctacaaaa atcattaccc gttca 25

<210> 6
<211> 25
<212> DNA
<213> Artificial Sequence

<220>
<223> Forward primer used for amplifying the dmrt1 gene

<400> 6
tgagagtggg tgtatgttat tgttt 25

<210> 7
<211> 25
<212> DNA
<213> Artificial Sequence

<220>
<223> Reverse primer used for amplifying the dmrt1 gene

<400> 7
actaacaatc cctccaatta caaaa 25

<210> 8
<211> 28
<212> DNA
<213> Artificial Sequence

<220>
<223> Forward primer used for amplifying the amhr-2 gene

<400> 8
gtgggaaatt ttttttatat ttttagga 28

<210> 9
<211> 25
<212> DNA
<213> Artificial Sequence

<220>
<223> Reverse primer used for amplifying the amhr-2 gene

<400> 9
acaacgacct aaacccttta ctaca 25

<210> 10
<211> 30
<212> DNA
<213> Artificial Sequence

<220>
<223> Forward primer used for the er-b2 gene in the MBS preparation

<400> 10
attatatttt tattttggta ttttttagtt 30

<210> 11
<211> 26
<212> DNA
<213> Artificial Sequence

<220>
<223> Reverse primer used for the er-b2 gene in the MBS preparation

<400> 11
accgacatta aaaattccaa cttcct 26

<210> 12
<211> 30
<212> DNA
<213> Artificial Sequence

<220>
<223> Forward primer used for the fshr gene in the MBS preparation

<400> 12
aatatagagg gaaataatag tgagagagtg 30

<210> 13
<211> 30

<212> DNA
<213> Artificial Sequence

<220>
<223> Reverse primer used for the fshr gene in the MBS preparation

<400> 13
aacaaaaact caaaattcgt ttaaccaaac 30

<210> 14
<211> 26
<212> DNA
<213> Artificial Sequence

<220>
<223> Forward primer used for the foxl2 gene in the MBS preparation

<400> 14
tagtttgtga ggatatgttt gagaag 26

<210> 15
<211> 25
<212> DNA
<213> Artificial Sequence

<220>
<223> Reverse primer used for the foxl2 gene in the MBS preparation

<400> 15
ttcccaataa aaacaataca tcatc 25

<210> 16
<211> 25
<212> DNA
<213> Artificial Sequence

<220>
<223> Forward primer used for the nr3c1 gene in the MBS preparation

<400> 16
ttattgtagg gattggagga ttaaa 25

<210> 17
<211> 25
<212> DNA
<213> Artificial Sequence

<220>
<223> Reverse primer used for the nr3c1 gene in the MBS preparation

<400> 17
accgctaact atcgatccaa taaca 25

<210> 18
<211> 33
<212> DNA
<213> Artificial Sequence

```
<220>
<223>  Forward primer used for the adapter in the MBS preparation

<400>  18
tcgtcggcag cgtcagatgt gtataagaga cag                                  33


<210>  19
<211>  34
<212>  DNA
<213>  Artificial Sequence

<220>
<223>  Reverse primer used for the adapter in the MBS preparation

<400>  19
gtctcgtggg ctcggagatg tgtataagag acag                                 34
```

## Description

The present invention relates to a method for predicting the sex in fish comprising determining the methylation levels of a set of epigenetic biomarkers. Thus, the present invention relates to the culture and breeding of aquatic animals, specifically, of fish.

## BACKGROUND ART

The integration of genomic and environmental information to bring about a given phenotype is a central area of current research on developmental epigenetics. Species where sex determination is dependent on both genetic and environmental influences provide excellent systems to address important questions such as how the environment shapes the epigenome during critical stages of early development with lifelong lasting consequences, and what is the contribution of epigenetic regulatory mechanisms into these processes. Thus, many poikilothermic vertebrates, i.e. species whose body temperature varies depending on the environment, exhibit environmental sex determination (ESD). This occurs in contrast to homoeothermic vertebrates, where sex is determined by a chromosomal system of genetic sex determination (GSD), either with male (XX/XY) or female (ZW/ZZ) heterogamety, as in mammals and birds, respectively. In species with ESD, sex is determined according to the magnitude of an environmental variable, of which temperature is the most relevant. ESD is present in many reptiles and in some fish. Fish, in particular, are known for having a remarkably plastic sex, and this applies not only to sequential hermaphrodites, which naturally undergo sex change as adults in response to external stimuli but, importantly, also to gonochoristic species, i.e., species where sex is separated in different individuals, exposed to abnormal conditions or to pollution. In fish, GSD includes species with sex chromosomes, where sex depends on the action of a "master" gene, not conserved even in closely related species, species with a major sex locus plus secondary loci, and species with polygenic sex determination (PSD), where many autosomal loci contribute with minor additive effects to sex determination. Consequently, GSD and ESD are nowadays regarded as the two ends of a continuum, where the sex of an individual can also be the result of both genetic and environmental influences, with transitions from one system to another occurring frequently. However, the underlying molecular mechanism linking temperature to sex ratios in poikilothermic vertebrates, including fish, but also reptiles, has been the subject of much debate. In this regard, PSD species offer a particularly attractive situation because, in a given batch of fertilized eggs, sex ratio is naturally dependent on both maternal and paternal influences (i.e., the "sex tendency" of each progenitor) and thus there will be variations in sex ratios across different families, although successive batches of the same parents will tend to exhibit similar sex ratios.

The European sea bass is one of those gonochoristic teleosts with PSD. At least three QTLs associated with sex determination have been identified in this species. Temperatures 13-16°C constitute the common range for spawning and early development. Variations in offspring sex ratios among families are observed. However, temperatures >17°C masculinize fish that under lower temperatures would develop as females. The thermosensitive period is 0-60 days post-fertilization (dpf). Like in other species, masculinization by elevated temperature involves downregulation of aromatase (cyp19a1) expression levels. Aromatase is the steroidogenic enzyme that irreversibly catalyzes the conversion of androgens to estrogens. Cyp19a1 expression and Cyp19a1 enzymatic activity are necessary to produce adequate amounts of estrogen, which are required for ovarian development in all vertebrates, except therian mammals. Thus, cyp19a1 is a key gene for sexual development in vertebrates. It was in the European sea bass where evidence of an epigenetic mechanism linking environmental temperature during early development and sex ratio was first described. Using bisulfite sequencing and a single gene approach targeting cyp19a1a, the gonadal isoform of aromatase, an inverse relationship between methylation of the cyp19a1a promoter and gene expression in females was found. Males have constitutively higher levels of cyp19a1a1 methylation than females. Masculinization results from high temperature-induced hypermethylation of cyp19a1a in females during early development, which prevents the binding of the transcription factor forkhead box L2 (foxl2) necessary for its transcriptional activation. This leads to a decrease of aromatase expression and the effect is carried out throughout adulthood. Similar results using a targeted single gene approach concerning the hypermethylation of the cyp19a1 promoter in male-producing temperatures were later reported in other sensitive vertebrates, including turtles, alligators and other fish, e.g. the olive flounder, so the role of aromatase as key actor in sexual development is well established. However, DNA methylation may also be positively correlated with gene expression under some situations. Thus, whether the relationship between cyp19a1 promoter methylation and gene expression holds across a wide range of methylation levels and is similar for both sexes is not clear. Furthermore, the methylation of other genes involved in sexual development might as well be affected by temperature. In this regard, in the European sea bass, even small temperature increases during early development are able to affect the DNA methylation of many genomic loci, as assessed by methylation-sensitive AFLP, a technique that does not allow single nucleotide resolution. Further, recent studies using whole genome bisulfite sequencing (WGBS) have shown that temperature can indeed affect many loci, as evidenced in *Nile tilapia.* In the half-smooth tongue sole, *Cynoglossus semilaevis,* a species with a ZW/ZZ chromosomal system with dmrt1 as the sex determining gene, WGBS revealed that the effects of elevated temperature on DNA methylation involved several genes related to sexual development such as dmrt1, amh-r2 and foxl2. In this species, fish with a ZW genotype (females) were sex-reversed into phenotypic males and their offspring even when reared at normal temperature still exhibited altered patterns of DNA methylation in the Z chromosome, suggesting an epigenetic transmission of altered DNA methylation patterns.

The Chinese patent application CN107217099 discloses a SNP marker for genetic sex and super male fish identification of snakehead. The patent application discloses a primer pair of the SNP marker used for the genetic sex and super male fish identification of the snakehead. The primer pair can amplify genomic DNA of a sample to be tested, can identify the genetic sex, and identify super male fishes from pseudo-female fish and ordinary male fish offspring families.

Nevertheless, there is still the need of alternative methods to those disclosed in the state of the art for determining the sex of fish in an accurate way.

## SUMMARY OF THE INVENTION

The authors of the present invention have discovered a set of epigenetic marks which allow the skilled person in the art to predict sex in fish with around 90% accuracy. These epigenetic marks relate to a set of methylation (CpGs) sites located at the *cyp19a1a* and *dmrt1* gene promoters which are differentially methylated in males and females. The location of CpG sites was carried out by specifically developed a cost-efficient multiplex bisulfite sequencing (MBS) approach for many samples to determine the methylation levels of cyp19a1a and dmrt1 genes (Example 1). Next, a Principal Component Analysis (PCA) was performed using the CpGs of these genes as variables (Example 2), showing that the average success rate for this method of predicting sex based only in the methylation information of the CpG of these genes was 88.54%.

Based on the above findings, the present invention relates to an *in vitro* method for predicting the sex in fish comprising

(a) Determining the methylation levels at the CpG sites of

(i) the *dmrt1* gene promoter within the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, wherein the CpG sites of dmrt1 gene promoter are at position 18,505,626, at position 18,505,637, at position 18,505,680, at position 18,505,688, at position 18,505,698, at position 18,505,732, at position 18,505,747, at position 18,505,791, at position 18,505,797, at position 18,505,890, at position 18,505,904, at position 18,505,945, at position 18,505,959, and at position 18,505,990, in a biological sample isolated from the fish, and

(ii) the *cyp19a1a* gene promoter within the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome, wherein the CpG sites of cyp19a1a gene promoter are at position 21,022,278, at position 21,022,271, at position 21,022,255, at position 21,022,235, and at position 21,022,214, and

(b) using the methylation levels determined in step (a) to obtain the probability of being a female, $F^P$, $(F^P = \frac{1}{1+e^{-P}})$ where

$$P = 0.4186 - 0.7749\,Y1 - 0.1297\,Y2$$

where

$$Y1 = \sum_{i=1}^{19} a_i \frac{(X_i - m_i)}{d_i}$$

and

$$Y2 = \sum_{i=1}^{19} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i,
wherein

- if $F^p > 0.5$ the fish is a female
- if $F^p < 0.5$ the fish is a male

## DETAILED DESCRIPION OF THE INVENTION

*Epigenetic biomarkers of the invention*

As explained above, the present invention provides a set of epigenetics biomarkers useful for predicting sex in fish, being said epigenetics biomarkers specific cytosine-guanine dinucleotides (CpGs) which are differentially methylated in males versus females. The inventors found out that the methylation levels of the promoters of the *dmrt1* and *cyp19a1a* genes were sufficient to group the fish by sex, wherein *cyp19a1a* gene is hypomethylated in ovaries with respect to testis, while *dmrt1* gene is hypomethylated in testis with respect to ovaries.

As used herein, the term "methylation" refers to cytosine methylation at positions C5 or N4 of cytosine. In vitro amplified DNA is unmethylated because in vitro DNA amplification methods do not retain the methylation pattern of the amplification template. However, "unmethylated DNA" or "methylated DNA" can also refer to amplified DNA whose original template was methylated or methylated, respectively. As used herein, the term "methylation level" as applied to a gene refers to whether one or more cytosine residues present in a CpG context have or do not have a methylation group. Methylation level may also refer to the fraction of cells in a sample that do or do not have a methylation group on such cytosines. Methylation level may also alternatively describe whether a single CpG di-nucleotide is methylated.

As used herein, the term "CpG" or "CG" site refers to cytosine and guanosine residues located sequentially (5'->3') in a polynucleotide DNA sequence.

As used herein the term "gene" refers to a genomic DNA sequence that comprises a coding sequence associated with the production of a polypeptide or polynucleotide product (e.g.,mRNA). The methylation level of a gene as used herein, encompasses the methylation level of sequences which are known or predicted to affect expression of the gene, including the promoter, enhancer, and transcription factor binding sites.

As used herein, the term "genome" refers to the complete set of genes or genetic material present in a cell or organism. The genome includes both the genes (the coding regions) and the noncoding DNA.

The genome location of CpG sites significantly relevant for determining the sex of fish, hereinafter "epigenetic biomarkers of the invention", is shown in Table 1 below:

**Table 1**. CpG sites location in the European sea bass genome. The genomic coordinates were calculated based on the version 1 (v1.0c) of the European sea bass genome (dicLab1), as annotated by the official public site of access to the genome (http://seabass.mpipz.de/) and accessed in August 2018. Alternatively, it is also shown the CpG position in the SEQ ID NO: 1 and SEQ ID NO: 2.

| Gene name | CpG site European sea bass genome | CpG (Chromosome:position) European sea bass genome |
|---|---|---|
| Doublesex- and mab-3-related transcription factor 1<br><br>Gene symbol: *dmrt1* | Position 46 of SEQ ID NO: 1 | LG20:18505626 |
| | Position 57 of SEQ ID NO: 1 | LG20:18505637 |
| | Position 74 of SEQ ID NO: 1 | LG20:18505680 |
| | Position 108 of SEQ ID NO: 1 | LG20:18505688 |
| | Position 118 of SEQ ID NO: 1 | LG20:18505698 |
| | Position 152 of SEQ ID NO: 1 | LG20:18505732 |
| | Position 167 of SEQ ID NO: 1 | LG20:18505747 |
| | Position 211 of SEQ ID NO: 1 | LG20:18505791 |
| | Position 217 of SEQ ID NO: 1 | LG20:18505797 |
| | Position 310 of SEQ ID NO: 1 | LG20:18505890 |
| | Position 324 of SEQ ID NO: 1 | LG20:18505904 |

(continued)

| Gene name | CpG site European sea bass genome | CpG (Chromosome:position) European sea bass genome |
|---|---|---|
| | Position 365 of SEQ ID NO: 1 | LG20:18505945 |
| | Position 379 of SEQ ID NO: 1 | LG20:18505959 |
| | Position 410 of SEQ ID NO: 1 | LG20:18505990 |
| Cytochrome P450 19A1a (Aromatase) | Position 388 of SEQ ID NO: 2 | LG5:21022278 |
| | Position 395 of SEQ ID NO: 2 | LG5:21022271 |
| | Position 411 of SEQ ID NO: 2 | LG5:21022255 |
| Gene symbol: *cyp19a1a* | Position 431 of SEQ ID NO: 2 | LG5:21022235 |
| | Position 452 of SEQ ID NO: 2 | LG5:21022214 |

SEQ ID NO: 1:

TAACTGAGAGTGGGTGCATGCCACTGCTCTGCCAGTTCTCTCCCCCGCTT
CCAGCTCGTTTCCTCCCACAAGTCGCAGCAGCAGCAGCAGCAGCAGCAGC
GGCAGGGCGGGGCAGGACGGTGACCTTATAGCCTCCAGCCTGGCACCACT
GCGAGCTTTAACCAGCCGAGCCTGCTGGGACAAACTCTAACATAGTAGCT
AAAGTCTGACCGCTGTCGTCTACCAGTTAGTGCACCAGACTTGTGTTGCA
GTTTTATTTCCAGCAGCCATGAGCAAGGACAAGCAGAGCAAGCAGGTGCC
AGAGTCCACCGGACCTCTGTCCCCGTCCAAAGGCCAGAAACCCCCCAGGA
TGCCCAAGTGTTCCCGCTGTAGGAACCACGGCTATGTGTCTCCTCTGAAG
GGACACAAGCGCTTTTGCAACTGGAGGGACTGTCAGTGTCCCAAA

SEQ ID NO: 1 shows the region around the *dmrt1* gene promoter on chromosome 20 from nucleotide 18,505,581 to 18,506,025 (LG20:18505581-18506025) of European sea bass genome version 1 (v1.0c) of the European sea bass genome (dicLab1), as annotated by the official public site of access to the genome (http://seabass.mpipz.de/) and accessed in August 2018.

SEQ ID NO: 2:

TATTCCAGCTTTCCGTTGTCTGTCTTCCTTTATAGATACAAAAGTAAATA
AAACTTGTGTGGATTTTTCTAGAAAAAAACTTCAATTCAATTTTGCAAGA
CAGATAAAGCTTTAACAATAATAATAACAATAACAGACTTTCCCATATTC
TCAGAGGCCAGATGTTCTTTTGTAGGGTTTCCAATCAGATTATTTTGATG
TTCAAAGGAGGAGCAAAACTTGCTTTTCATCACTAAAATGTCAAATAACC
CCACTCCAACTCCAACATGTACTCACTGAACTAAGTCCTGTACTCTCAAG
AGCACAGGCACTAATACAACCCTTCAAGGTTACAAGTGTATTGTTTACCA
TTTTTCTCCTCTGTTGTGGCTTTTACTTTGCCCTGACGTGGCTCGTAACC
AGCTCAGACCGCATATAAAGAGAAAGCCCCGATTGTTGAGGCAGCTCACA
CGGAGCAGTTGCTTTTGGTTATTTTAAATGGATCTGATCTCTGCATGTGA

ACGGGCAATGACTCCTGTAGGTT

SEQ ID NO: 2 shows the region around the *cyp19a1a* gene promoter on chromosome 5 from nucleotide 21,022,143 to 21,022,665 (LG5:21022143-21022665) of European sea bass genome version 1 (v1.0c) of the European sea bass genome (dicLab1), as annotated by the official public site of access to the genome (http://seabass.mpipz.de/) and accessed in August 2018.

Thus, in an aspect, the present invention relates to the use of the epigenetic biomarkers of the invention for predicting the sex in fish.

Additionally, the inventors also found that in order to increase the accurate of the prediction, the methylation levels of further epigenetic markers located in said genes (cyp19a1a) and additional gene (Amh-r2) can also be determined. Thus, in a particular embodiment, the epigenetic biomarkers of the invention further comprise one, two and/or three of the markers shown in Table 2 below:

Table 2. CpG sites location in the European sea bass genome. The genomic coordinates were calculated based on the version 1 (v1.0c) of the European sea bass genome (dicLab1), as annotated by the official public site of access to the genome (http://seabass.mpipz.de/) and accessed in August 2018. Alternatively, it is also shown the CpG position in the SEQ ID NO: 2 and SEQ ID NO: 3.

| Gene name | CpG site | CpG (Chromosome:position) European sea bass genome |
|---|---|---|
| *cyp19a1a* gene | Position 15 of SEQ ID NO: 2 | LG5:21022651 |
| *Amh-r2* gene | Position 94 of SEQ ID NO: 3 | LG1A:20275623 |
| | Position 208 of SEQ ID NO: 3 | LG1A:20275509 |
| | Position 293 of SEQ ID NO: 3 | LG1A:20275424 |

SEQ ID NO: 3

```
GTGGGAAACTCCTCTCACATTCCCAGGAATGACAAATATTCACTCCCTAC
TTGGCTGGTCTGGACTCAGAGTGACCATGTTCCTGCAACAGTGGCGACTG
ATTTTGGCTTTGGGTGAGTGCATTTGCTCTTAAAAAAACCCCTCTGTTTC
TTGTGTTTCTTTCTATTTTGTTTTACATTTCAATCTTAAAATGAAAGACT
TAGATATGCGTGTTTTGCTTGGATAGTGACAGTAGAGAGACACAGGAAAG
TCAGGGGAGAGAGAGGGGATGACTTGCAGCAAAGGGTCCAGGCCGCTGTG
T
```

SEQ ID NO: 3 shows the region around the *amh-r2* gene promoter on chromosome 1A from nucleotide 20,275,417 to nucleotide 20,275,717 (LG1A:20275417-20275717) of European sea bass genome version 1 (v1.0c) of the European sea bass genome (dicLab1), as annotated by the official public site of access to the genome (http://seabass.mpipz.de/) and accessed in August 2018.

*Method of the invention*

In one aspect, the present invention relates to an *in vitro* method for predicting the sex in fish, hereinafter "method of the invention", said method comprising

(a) Determining the methylation levels at the CpG sites of

(i) the *dmrt1* gene promoter within the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, wherein the CpG sites of dmrt1 gene promoter are at position 18,505,626, at position 18,505,637, at position 18,505,680, at position 18,505,688, at position 18,505,698, at position 18,505,732, at position 18,505,747, at position 18,505,791, at position 18,505,797, at position 18,505,890, at position 18,505,904, at position 18,505,945, at position 18,505,959, and at position 18,505,990, in a biological sample isolated from the fish, and

(ii) the *cyp19a1a* gene promoter within the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome, wherein the CpG sites of cyp19a1a gene promoter are at position 21,022,278, at position 21,022,271, at position 21,022,255, at position 21,022,235, and at position 21,022,214, and

(b) using the methylation levels determined in step (a) to obtain the probability of being a female, $F^P$, $(F^P = \frac{1}{1+e^{-P}})$

where

$$P = 0.4186 - 0.7749\,Y1 - 0.1297\,Y2$$

where

$$Y1 = \sum_{i=1}^{19} a_i \frac{(X_i - m_i)}{d_i}$$

and

$$Y2 = \sum_{i=1}^{19} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i.
wherein

- if $F^p > 0.5$ the fish is a female
- if $F^p < 0.5$ the fish is a male

In a first step, the method of the invention comprises determining the methylation levels at the CpG sites of the *dmrt1* gene promoter and the *cyp19a1a* gene promoter, i.e. the methylation levels of the epigenetic biomarkers of the invention as defined above.

Any method for determining or detecting methylation levels can be used in the methods of the present invention. Examples of methods for determining methylation levels at the CpG sites include, but are not limited to, bisulfite sequencing, pyrosequencing, methylation-sensitive single nucleotide primer extension (MS-SnuPE),, methylation-specific PCR (MSP), Sanger bisulfite sequencing, Bisulfite Restriction Site Associated DNA (BisRAD), methylation-sensitive restriction enzyme-based methods, microarray-based methods, whole-genome bisulfite sequencing (WGBS, MethylC-seq or BS-seq), reduced-representation bisulfite sequencing(RRBS), and/or enrichment-based methods such as MeDIP-seq, MBD-seq, or MRE-seq when combined with bisulfite conversion.

In some embodiments, methods for detecting methylation levels include randomly shearing or randomly fragmenting the genomic DNA, cutting the DNA with a methylation-dependent or methylation-sensitive restriction enzyme and subsequently selectively identifying and/or analyzing the cut or uncut DNA.

A "methylation-dependent restriction enzyme" refers to a restriction enzyme that cleaves or digests DNA at or in proximity to a methylated recognition sequence, but does not cleave DNA at or near the same sequence when the recognition sequence is not methylated. Methylation-dependent restriction enzymes include those that cut at a methylated recognition sequence and enzymes that cut at a sequence near but not at the recognition sequence. Exemplary methylation-dependent restriction enzymes include, but are not limited to, McrBC, McrA, MrrA, BisI, GlaI and DpnI. One of skill in the art will appreciate that any methylation-dependent restriction enzyme, including homologs and orthologs of the restriction enzymes described herein, is also suitable for use in the present invention.

A "methylation-sensitive restriction enzyme" refers to a restriction enzyme that cleaves DNA at or in proximity to an unmethylated recognition sequence but does not cleave at or in proximity to the same sequence when the recognition sequence is methylated. Exemplary methylation-sensitive restriction enzymes that do not cleave DNA at or near their recognition sequence when a cytosine within the recognition sequence is methylated include, without limiting to, Aat II, Aci I, Acl I, Age I, Alu I, Ase I, Ase I, AsiS I, Bbe I, BsaA I, BsaH I, BsiE I, BsiW I, BsrF I, BssH II, BssK I, BstB I, BstN I, BstU I, Cla I, Eae L, Eag L, Fau I, Fse I, Hha I, HinP1 I, HinC II, Hpa II, Hpy99 I, HpyCH4 IV, Kas I, Mbo I, Mlu I, MapA1 I, Msp I, Nae I, Nar I, Not I, Pml I, Pst I, Pvu I, Rsr II, Sac II, Sap I, Sau3A I, Sfl I, Sfo I, SgrA I, Sma I, SnaB I, Tsc I, Xma I, and Zra I. One of skill in the art will appreciate that any methylation-sensitive restriction enzyme, including homologs and orthologs of the restriction enzymes described herein, is also suitable for use in the present invention. One of skill in the art will further appreciate that a methylation-sensitive restriction enzyme that fails to cut in the presence of methylation of a cytosine at or near its recognition sequence may be insensitive to the presence of methylation of an adenosine at or near its recognition sequence. For example, Sau3AI is sensitive (i.e., fails to cut) to the presence of a methylated cytosine at or near its recognition sequence, but is insensitive (i.e., cuts) to the presence of a methylated adenosine at or near its recognition sequence. One of skill in the art will also appreciate that some methylation-sensitive restriction enzymes are blocked by methylation of bases on one or both strands of DNA encompassing of their recognition sequence, while other methylation-sensitive restriction enzymes are blocked only by methylation on both strands, but can cut if a recognition site is hemi-methylated.

Selective identification can include, for example, separating cut and uncut DNA (e.g., by size) and quantifying a sequence of interest that was cut or, alternatively, that was not cut. Alternatively, the method can encompass amplifying intact DNA after restriction enzyme digestion, thereby only amplifying DNA that was not cleaved by the restriction enzyme in the area amplified. In some embodiments, amplification can be performed using primers that are gene specific. Alternatively, adaptors can be added to the ends of the randomly fragmented DNA, the DNA can be digested with a methylation-dependent or methylation-sensitive restriction enzyme, intact DNA can be amplified using primers that hybridize to the adaptor sequences. In this case, a second step can be performed to determine the presence, absence or quantity of a particular gene in an amplified pool of DNA. In some embodiments, the DNA is amplified using real-time, quantitative PCR.

In some embodiments, the methods comprise quantifying the average methylation density in a target sequence within a population of genomic DNA. In some embodiments, the method comprises contacting genomic DNA, with a methylation-dependent restriction enzyme or methylation-sensitive restriction enzyme under conditions that allow for at least some copies of potential restriction enzyme cleavage sites in the locus to remain uncleaved; quantifying intact copies of the locus; and comparing the quantity of amplified product to a control value representing the quantity of methylation of control DNA, thereby quantifying the average methylation density in the locus compared to the methylation density of the control DNA.

The methylation level of a CpG site can be determined by providing a sample of genomic DNA comprising the CpG locus, cleaving the DNA with a restriction enzyme that is either methylation-sensitive or methylation-dependent, and then quantifying the amount of intact DNA or quantifying the amount of cut DNA at the locus of interest. The amount of intact or cut DNA will depend on the initial amount of genomic DNA containing the locus, the amount of methylation in the locus, and the number (i.e., the fraction) of nucleotides in the locus that are methylated in the genomic DNA. The amount of methylation in a DNA locus can be determined by comparing the quantity of intact DNA or cut DNA to a control value representing the quantity of intact DNA or cut DNA in a similarly-treated DNA sample. The control value can represent a known or predicted number of methylated nucleotides. Alternatively, the control value can represent the quantity of intact or cut DNA from the same locus in another fish.

By using at least one methylation-sensitive or methylation-dependent restriction enzyme under conditions that allow for at least some copies of potential restriction enzyme cleavage sites in the locus to remain uncleaved and subsequently quantifying the remaining intact copies and comparing the quantity to a control, average methylation density of a locus can be determined. If the methylation-sensitive restriction enzyme is contacted to copies of a DNA locus under conditions that allow for at least some copies of potential restriction enzyme cleavage sites in the locus to remain uncleaved, then the remaining intact DNA will be directly proportional to the methylation density, and thus may be compared to a control to determine the relative methylation density of the locus in the sample. Similarly, if a methylation-dependent restriction enzyme is contacted to copies of a DNA locus under conditions that allow for at least some copies of potential restriction enzyme cleavage sites in the locus to remain uncleaved, then the remaining intact DNA will be inversely proportional to the methylation density, and thus may be compared to a control to determine the relative methylation density of the locus in the sample.

Kits for the above methods can include, e.g., one or more of methylation-dependent restriction enzymes, methylation-sensitive restriction enzymes, amplification (e.g., PCR) reagents, probes and/or primers.

Quantitative amplification methods (e.g., quantitative PCR or quantitative linear amplification) can be used to quantify the amount of intact DNA within a locus flanked by amplification primers following restriction digestion. Amplifications may be monitored in "real time."

Additional methods for detecting methylation levels can involve genomic sequencing before and after treatment of the DNA with bisulfite. When sodium bisulfite is contacted to DNA, unmethylated cytosine is converted to uracil, while methylated cytosine is not modified. Such additional embodiments include the use of array-based assays and multiplex PCR assays. The multiplex PCR assay may be Patch PCR. PatchPCR can be used to determine the methylation level of a certain CpG loci. Bisulfite Patch PCR enables multiplexed sequencing of promoter methylation across cancer samples.

Alternatively, restriction enzyme digestion of PCR products amplified from bisulfite-converted DNA is used to detect DNA methylation levels. A "MethyLight" assay may be used alone or in combination with other methods to detect methylation level. Briefly, in the MethyLight process, genomic DNA is converted in a sodium bisulfite reaction (the bisulfite process converts unmethylated cytosine residues to uracil). Amplification of a DNA sequence of interest is then performed using PCR primers that hybridize to CpG dinucleotides. By using primers that hybridize only to sequences resulting from bisulfite conversion of unmethylated DNA, (or alternatively to methylated sequences that are not converted) amplification can indicate methylation status of sequences where the primers hybridize. Similarly, the amplification product can be detected with a probe that specifically binds to a sequence resulting from bisulfite treatment of an unmethylated (or methylated) DNA. If desired, both primers and probes can be used to detect methylation status. Thus, kits for use with MethyLight can include sodium bisulfite as well as primers or detectably-labeled probes (including but not limited to Taqman or molecular beacon probes) that distinguish between methylated and unmethylated DNA that have been treated with bisulfite. Other kit components can include, e.g., reagents necessary for amplification of DNA including but not limited to, PCR buffers, deoxynucleotides; and a thermostable polymerase.

A Ms-SNuPE (Methylation-sensitive Single Nucleotide Primer Extension) reaction may also be used in the present invention, well alone or in combination with other methods to detect methylation level. The Ms-SNuPE technique is a quantitative method for assessing methylation differences at specific CpG sites based on bisulfite treatment of DNA, followed by single-nucleotide primer extension. Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest.

Typical reagents (e.g., as might be found in a typical Ms-SNuPE-based kit) for Ms-SNuPE analysis can include, but are not limited to: PCR primers for specific gene (or methylation-altered DNA sequence or CpG island); optimized PCR buffers and deoxynucleotides; gel extraction kit; positive control primers; Ms-SNuPE primers for a specific gene; reaction buffer (for the Ms-SNuPE reaction); and detectably-labeled nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

In a particular embodiment of the present invention, the primers used are shown in Table 3 below.

Table 3. Primers used for amplifying the DNA region comprising the epigenetic biomarkers of the invention. The genomic coordinates were calculated based on the version 1 (v1.0c) of the European sea bass genome (dicLab1), as annotated by the official public site of access to the genome (http://seabass.mpipz.de/) and accessed in August 2018.

| Gene symbol | | Primer (5' to 3') | Genomic position in the European sea bass genome (Chromosome: position) |
|---|---|---|---|
| *cyp19a1a* | Forward | TTAGTTTTTCGTTGTTTGTTTTT (SEQ ID NO: 4) | LG5: 21022143 - 21022665 |
| | Reverse | ACCTACAAAAATCATTACCCGTTCA (SEQ ID NO: 5) | |
| *dmrt1* | Forward | TGAGAGTGGGTGTATGTTATTGTTT (SEQ ID NO: 6) | LG20: 18505581 - 18506025 |
| | Reverse | ACTAACAATCCCTCCAATTACAAAA (SEQ ID NO: 7) | |
| *amhr-2* | Forward | GTGGGAAATTTTTTTTATATTTTTAGGA (SEQ ID NO: 8) | LG1A:20275417 -20275717 |
| | Reverse | ACAACGACCTAAACCCTTTACTACA (SEQ ID NO: 9) | |

A methylation-specific PCR ("MSP") reaction may also be used alone or in combination with other methods to detect DNA methylation. An MSP assay entails initial modification of DNA by sodium bisulfite, converting all unmethylated, but not methylated, cytosines to uracil, and subsequent amplification with primers specific for methylated versus unmethylated DNA.

In a particular embodiment, step (a) of the method of the invention comprises the steps of:

(i) treating genomic DNA obtained from a sample with the compound modifying an unmethylated cytosine base or the methylation sensitive restriction enzyme; and
(ii) measuring the methylation level of the treated DNA by one or more methods selected from the group consisting of methylation-specific polymerase chain reaction, real time methylation-specific polymerase chain reaction, PCR using a methylated DNA-specific binding protein, quantitative PCR, pyrosequencing and bisulfite sequencing using primers capable of amplifying the methylated region at the CpG site of the gene.

In another particular embodiment of the method of the invention, the compound modifying an unmethylated cytosine base is bisulfite or a salt thereof.

As used herein, the term "treating DNA from the sample with bisulfite" refers to treatment of DNA with a reagent comprising bisulfite, disulfite, hydrogen sulfite or combinations thereof, for a time and under conditions sufficient to convert unmethylated DNA cytosine residues to uracil, thereby facilitating the identification of methylated and unmethylated CpG dinucleotide sequences. Bisulfite modifications to DNA may be detected according to methods known in the art, for example, using sequencing or detection probes which are capable of discerning the presence of a cytosine or uracil residue at the CpG site.

The method of the invention comprises detecting the methylation levels of the *dmrt1* gene promoter at the CpC sites within a DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome.

The DNA region of the *dmrt1* gene promoter as well as the sequence SEQ ID NO: 1 have been defined in previous paragraphs regarding the epigenetic biomarkers of the invention. As used herein, the term "identity" or "sequence identity" is understood to mean the degree of similarity between two nucleotide or amino acid sequences obtained by aligning the two sequences. Depending on the number of common residues between the aligned sequences, a different degree of identity, expressed as a percentage, will be obtained. Nucleotide sequences are said to be homologous when exhibiting a certain degree of identity. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programmes, for example, BLASTN, BLASTX, and T BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality.

In a more particular embodiment, the DNA region of the dmrt1 gene promoter comprises a nucleotide sequence having a sequence identity of at least 90, 91, 92, 93, 94, 95, 96, 97, 98 o 99% to the SEQ ID NO: 1, as long as it comprises the epigenetic biomarkers of the invention. In an even more particular embodiment, the DNA region of the dmrt1 gene promoter comprises a nucleotide sequence having a sequence identity of 100% to the SEQ ID NO: 1.

Furthermore, the method of the invention also comprises detecting the methylation levels of the *cyp19a1a* gene promoter at the GpC sites within DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome.

In a more particular embodiment, the DNA region of the *cyp19a1a* gene promoter comprises a nucleotide sequence having a sequence identity of at least 90, 91, 92, 93, 94, 95, 96, 97, 98 o 99% to the SEQ ID NO: 2, as long as it comprises the epigenetic biomarkers of the invention. In an even more particular embodiment, the DNA region of the *cyp19a1a* gene promoter comprises a nucleotide sequence having a sequence identity of 100% to the SEQ ID NO: 2.

The method of the invention may comprise detecting the methylation levels of additional epigenetic biomarkers in order to increase the accuracy of the method. Thus, in a particular embodiment, step (a) of the method of the invention, further comprises determining the methylation levels of the CpG site of *cyp19a1a* gene promoter at position 21,022,651 of the chromosome 5 of the European seabass genome, and step b) comprises using the methylation levels determined in step (a) to obtain the probability of being a female, $F^P$, $(F^P = \frac{1}{1+e^{-P}})$ where P = 0.4176 - 0.7755 Y1 - 0.1298 Y2 where

$$Y1 = \sum_{i=1}^{20} a_i \frac{(X_i - m_i)}{d_i}$$

and

$$Y2 = \sum_{i=1}^{20} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i,
wherein

- if $F^p > 0.5$ the fish is a female
- if $F^p < 0.5$ the fish is a male.

In another particular embodiment, step (a) of the method of the invention, further comprises determining the methylation levels at CpG sites of *amh-r*2 gene promoter within the DNA region comprising a nucleotide sequence having a sequence identity of at least 90% to the DNA region from nucleotide 20,275,417 to nucleotide 20,275,717 (SEQ ID NO: 3) of the chromosome LG1A of the European seabass genome, wherein

- the CpG sites of amh-r2 gene promoter are at position 20,275,424, at position 20,275,509 and/or at position 20,275,623, and
- using the methylation levels determined in step (a) to obtain the probability of being a female, $F^P$, $(F^P = \frac{1}{1+e^{-P}})$ where

$$P = = 0.4397 + 0.7869\,Y1 - 0.1672\,Y2$$

where

$$Y1 = \sum_{i=1}^{23} a_i \frac{(X_i - m_i)}{d_i}$$

and

$$Y2 = \sum_{i=1}^{23} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i.
wherein

- if $F^p > 0.5$ the fish is a female
- if $F^p < 0.5$ the fish is a male

As explained at the beginning of the present description, the present invention is based on the findings that *cyp19a1a* gene is hypomethylated in ovaries with respect to testis, while *dmrt1* gene is hypomethylated in testis with respect to ovaries. Thus, in order to predict the sex of the fish based on the methylation levels of said genes, the reference value is the methylation levels of said genes in a group of male and female fish.

In order to put into practice the method of the invention, it is necessary to determine the methylation levels of the epigenetic biomarkers of the invention in a biological ample of a fish. As used herein, the term "biological sample" refers to any sample which can be obtained and isolated from the subject and comprises DNA. The term "isolated" means a substance in a form or in an environment that does not occur in nature. The present method can be applied to a biological sample from a fish, such as a biopsy sample or tissue. In a particular embodiment, said sample is tissue sample, more particularly, gonads tissue. A tissue sample can be obtained by conventional methods, e.g., biopsy, by using methods well known to those of ordinary skill. Methods for obtaining the sample from the biopsy include gross apportioning of a mass, or microdissection or other art-known cell-separation methods.

Once the biological sample has been obtained, the DNA has to be isolated. Methods for isolating DNA from a sample are widely known in the state of the art and are routine practice for the skilled person.

The method of the invention can be applied to any fish. In a particular embodiment, the fish is a bass, preferably, the fish is a European sea bass (*Dicentrarchus labrax*), spotted European sea bass (*Dicentrarchus punctatus*), striped bass (*Morone saxatilis*), white bass (*Morone chrysops*), largemouth bass (*Micropterus salmoides*), redeye bass (*Micropterus coosae*), spotted bass (*Micropterus punctulatus*), shoal bass (*Micropterus cataractae*), smallmouth bass (*Micropterus dolomieu*), choctaw bass (*Micropterus haiaka*), Japanese seabass (*Lateolabrax japonicus*), or blackfin sea bass (*Lateolabrax latus*).

Alternative to the above-disclosed method, the present invention also relates to the use of the methylation levels of the epigenetic biomarkers of the invention for determining the sex of fish.

Therefore, in another aspect, the present invention relates to the use of the methylation levels at the CpG sites of the *dmrt1* gene promoter and of the *cyp19a1a* gene promoter for determining the sex of fish, hereinafter "use of the invention", wherein

- the CpG sites of *dmrt1* gene promoter are at position 18,505,626, at position 18,505,637, at position 18,505,680, at position 18,505,688, at position 18,505,698, at position 18,505,732, at position 18,505,747, at position 18,505,791, at position 18,505,797, at position 18,505,890, at position 18,505,904, at position 18,505,945, at position 18,505,959, and at position 18,505,990 from the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, and
- the CpG sites of *cyp19a1a* gene promoter are at position 21,022,278, at position 21,022,271, at position 21,022,255, at position 21,022,235, and at position 21,022,214 from the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome.

In a particular embodiment, the use of the invention further comprises

- the methylation levels of the CpG site of *cyp19a1a* gene promoter at position 21,022,651 of the chromosome 5 of the European seabass genome, and/or
- the methylation levels of the CpG sites of *amh-r2* gene promoter at position 20,275,424, at position 20,275,509 and/or at position 20,275,623 from the DNA region comprising a nucleotide sequence having a sequence identity of at least 90% to the DNA region from nucleotide 20,275,417 to nucleotide 20,275,717 (SEQ ID NO: 3) of the chromosome LG1A of the European seabass genome.

All the definitions and particular embodiments disclosed for the method of the invention are applicable to the use of the invention.

*Kit of the invention*

The present invention also provides kits for determining the methylation levels of the epigenetics biomarkers of the invention, so that it is possible to predict the sex of fish as explained in the beginning of the present disclosure.

Thus, in another aspect, the present invention relates to a kit, hereinafter "kit of the invention", comprising nucleic acid primers specifically designed to hybridize with the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, and nucleic acid primers specifically designed to hybridize with the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome.

In another particular embodiment, the kit of the invention further comprises nucleic acid primers specifically designed to hybridize with the DNA region comprising a nucleotide sequence having a sequence identity of at least 90% to the DNA region from nucleotide 20,275,417 to nucleotide 20,275,717 (SEQ ID NO: 3) of the chromosome LG1A of the European seabass genome.

In another particular embodiment of the kit of the invention comprises the pair of primers SEQ ID NO: 4 and SEQ ID NO: 5, and/or SEQ ID NO: 6 and SEQ ID NO: 7.

Reagents for detection of methylation include, e.g., sodium bisulfite, polynucleotides designed to hybridize to sequence that is the product of a biomarker sequence of the invention if the biomarker sequence is not methylated, and/or a methylation-sensitive or methylation-dependent restriction enzyme. The kits can provide solid supports in the form of an assay apparatus that is adapted to use in the assay. The kits may further comprise detectable labels, optionally linked to a polynucleotide, e.g., a probe, in the kit. Other materials useful in the performance of the method can also be included in the kits, including test tubes, transfer pipettes, and the like. The kits can also include written instructions for the use of one or more of these reagents in any of the assays described herein.

In some embodiments, the kit of the invention comprises one or more (e.g., 1, 2, 3, 4, or more) different polynucleotides (e.g., primers) capable of specifically amplifying at least a portion of a DNA region where the DNA region includes the epigenetic biomarkers of the invention. Optionally, one or more detectably-labeled polypeptides capable of hybridizing to the amplified portion can also be included in the kit. In some embodiments, the kits comprise sufficient primers to amplify 2, 3, 4, 5, 6, 7, 8, 9, 10, or more different DNA regions or portions thereof, and optionally include detectably-labeled polynucleotides capable of hybridizing to each amplified DNA region or portion thereof. The kits further can comprise a methylation-dependent or methylation sensitive restriction enzyme and/or sodium bisulfite.

In another aspect, the present invention relates to the use of the kit of the invention for determining the sex of fish.

In a particular embodiment, the fish is a bass, preferably, the fish is a European sea bass (*Dicentrarchus labrax*), spotted European sea bass (*Dicentrarchus punctatus*) striped bass (*Morone saxatilis*), white bass (*Morone chrysops*), largemouth bass (*Micropterus salmoides*), redeye bass (*Micropterus coosae*), spotted bass (*Micropterus punctulatus*), shoal bass (*Micropterus cataractae*), smallmouth bass (*Micropterus dolomieu*), choctaw bass (*Micropterus haiaka*), Japanese seabass (*Lateolabrax japonicus*), or blackfin sea bass (*Lateolabrax latus*).

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

## BRIEF DESCRIPTION OF THE DRAWINGS

**Figure 1. Experimental set-up and sex ratios of the offspring.** A) Experimental set-up of crossings and temperature treatments. Eggs were obtained from crossing sires known from previous trials to produce offspring with lower (sires a and b) or higher (sires c and d) percentage of females. The sires were crossed with two females, however the offspring of second female only represented the 0.9% and was subsequently discarded from further analysis. Two days post fertilization (dpf) eggs were mixed according to the male prone and female prone groups. On day 13, larvae from the two groups were divided into four with half of the fish being raised at low temperature (LT; 16.5°C) and the other half at high temperature (HT; 21°C) until 65 dpf, the end of the thermosensitive period. In each tank, an equal number of albino fish was added as a control for tank effects. Fish were sampled at one year of age (323 dpf) and gonad samples were taken from 10 females and 10 males for the LT groups and 10 females and 20 males from the HT groups from each tank. B) Paternal effects on European sea bass sex ratio and effects of elevated temperature. Percent of female (red) and male (blue) offspring of each sire (a, b, c and d) raised at low (LT) or high (HT) temperature. Absolute numbers of analyzed fish are shown in the bottom of each bar. The dotted lines point out project the sex ratio percent from the LT to the HT offspring and vice versa. The numbers between the dotted lines inside the LT bars indicate the percent of sex-reversed females among the LT females, while the numbers inside the HT bars indicate the percent of presumed neomales (sex-reversed females into males) among the HT males. The effects of temperature on sex ratios were assessed by Fisher's exact test for count data and shown with the following equivalence: ** = $p<0.01$.

**Figure 2. Sex ratio of experimental and albino fish.** Datapoint symbols indicate the percent of females of experimental and albino fish in the same tank and circles surround the two replicate tanks for each experimental group: offspring of sires a and b (circles) at low (LT; blue) and high (HT; red) temperature and offspring of sires c and d (squares) at low (blue) and high (red) temperature. The average female percent in the albino fish reared at both low and high temperature was 45.63% and their sex ratio not different from 1:1.

**Figure 3. European sea bass growth as a function of genetic and environmental factors.** A) Body weight, B) fork length, C) sex dimorphic growth and D) condition factor, according to sire (a, b, c and d), sex and temperature (LT, low; HT, high). In A, B and D data is shown as mean $\pm$ S.E.M. In C the double datapoints per sire and temperature refer to the replicate tanks. Refer to S2 Table for details on statistical significance as assessed by a multifactorial ANOVA.

**Figure 4. DNA methylation levels of the genes examined in the parents and the offspring.** In the left part of the figure, the DNA methylation in the oocytes of the dam (grey), the sperm of sire a (deep pink), b (violet), c (orange) and d (green blue) is shown. The central and right part of the figure illustrate the DNA methylation levels in the offspring, separately by each sire with the background color indicating the corresponding sire and also, in the offspring of the four sires combined. The offspring is divided in four groups according to sex and temperature experienced during early development and DNA methylation values are shown in the ovaries of low (yellow) and high (red) temperature females and in the testis of low (light blue) and high (blue) temperature males. The far-right data indicate p-values for the effects of sex (S), temperature (T) or their interaction (SxT). The absolute numbers of fish analyzed in each case are shown inside the bars. Data as mean ± SEM.

**Figure 5. Correlations of DNA methylation among the different CpGs of the *cyp19a1a* promoter.** Correlations of DNA methylation are shown for the CpG at position 21022651 vs. the rest CpGs (A), the CpG at position 21022163 vs. the rest CpGs excluding the position 21022651 (B), the CpG at position 21022214 vs. the rest CpGs excluding positions 21022651 and 21022163, and the CpG at position 21022278 vs the CpGs at positions 21022271, 21022255 and 21022235. Pearson's product-moment correlation coefficients (ρ) and p-values of correlation significance are shown.

**Figure 6. Pairwise correlations of methylation levels of the 7 CpGs of the *cyp19a1a* promoter and first exon.** In the diagonal cells, the positions of the CpG relative to the transcription start site (TSS) is shown, from 21022651 to 21022163. The cells below the diagonal display scatterplots of the mean methylation of the vertical CpG (x-axis) and the horizontal CpG (y-axis) per temperature (L, low and H, high temperature), sire (a, b, c, d) and sex (F, females indicated by circles and M, males by squares). Loess smoothers are shown in red in each scatterplot. Pearson's correlation coefficients are displayed in the cells above the diagonal with the significance level of the correlation denoted (*** =$p$<0.001).

**Figure 7. Methylation differences from sire to offspring in individual CpGs.** Mean methylation difference in individual CpGs of the seven genes analyzed between the sperm and the gonads of their male and female offspring reared at low (control) temperature. Information is provided individually for sires a-d (A) and independently of sire (B). Data are shown as the mean of methylation differences of the corresponding sire to the individual fish in each group ± SEM.

**Figure 8. Relationship of methylation in the sires and in the offspring.** Scatterplot of the mean methylation differences per gene calculated as levels in the sires minus levels in their corresponding offspring reared at low temperature (female offspring: circles; male offspring: squares). Methylation differences close to zero indicate stable methylation levels, whereas differences above and below zero indicate hypomethylation and hypermethylation, respectively, in offspring vs. parents. The regression lines correspond to genes with low methylation differences between sires and offspring (*foxl2, nr3c1* and *er-β2*) and genes with higher methylation differences between sires and offspring (*cyp19a1a, dmrt1, fsh-r* and *amh-r2*).

**Figure 9. Methylation differences between high and low temperature in individual CpGs.** Mean methylation difference in individual CpGs of the seven genes analyzed between the offspring reared at high vs. low temperature according to sex. Information is provided individually for sires a-d (A) and independently of sire (B). Data are shown as the difference of the mean methylation of individual fish reared at high temperature minus the mean methylation of individual fish reared at low temperature.

**Figure 10. Methylation differences between fish reared at low and high temperature.** Scatterplot of the mean methylation differences per gene calculated as levels in offspring reared at high temperature minus levels in offspring reared at low temperature offspring reared at low temperature. The value mean of these differences is plotted against the corresponding mean methylation of female (circles) and male (squares) fish reared at low temperature (A) and high temperature (B).

**Figure 11. Prediction of offspring phenotypic sex using the methylation of selected CpGs as epigenetic biomarkers.** A) The DNA methylation levels of individual CpGs from the three genes that presented the highest differences between fish reared at low and at high temperature were the multiple variables used in the PCA. The individual fish are plotted as dots in the space of the two principal components. The percentage of variance explained by the two first components is shown in parenthesis. Of the total sample size available (n=87), 83% (n=72) were used as training set and are colored according to sex (female, red; male, blue) and 17% (n=15) of the individual fish are colored in green for which the coordinates and hence the sex was predicted based on the training set (F, females; M, males). Confidence ellipses are drawn for the two groups and colored according to sex. The pink arrows point to the two predicted individuals for which prediction of sex failed. The names of the variables (Bn and Cn; n = 1, 2, ...) correspond to the CpGs of informative genes. Of the total CpGs used for the PCA only the 10 with the highest contribution to the principal components are shown for clarity.

Figure 12. The overall relationship between dmrt1 and cyp19a1a of representative values at each CpG are in Figure 12 for males and females, although absolute values can vary depending on biological samples of origin.

**Figure 13. Prediction of sex in a sample.** Following the procedures described, the sample fish position in the plot indicates that is a female, corrobotaing that $F^p > 0.5$. Symbols as in Figure 11.

## EXAMPLES

### Example 1

I. MATERIAL AND METHODS

#### Fish and general rearing conditions

Four West Mediterranean European sea bass sires (sires a-d) were selected based on their tendency to produce more or less females in the offspring, as assessed in two preliminary crossings. Thus, sire a had given 0% and 20.5% females (mean: 10.25%); sire b, 7.5 and 8.8% females (mean: 8.15); sire c, 25.0% and 43.2% females (mean: 34.1); and sire d, 25.9% and 58.6% females (mean: 42.25%). We crossed each sire with two randomly chosen West Mediterranean dams, thus producing 8 families. Cryopreserved sperm from the four sires and eggs from the two dams were kept for evaluation of the DNA methylation analysis. After fertilization, the batches corresponding to the four sires were incubated separately at ~14.5°C, then equalized in volume of floating eggs at 48 hours post fertilization and mixed in two groups, a "male-prone" group, containing the offspring of sires a and b, and a "female-prone" group, containing the offspring of sires c and d (Figure 1A). A second crossing of eleven dams and twenty albino sea bass sires was done the same day of the experimental crossing. These fish were used as "spike-ins" to control for possible tank effects on sex ratio (see below). All procedures performed were in accordance with the ethical standards of the institution and followed the European Directive 2010/63 UE. All fish handling procedures were conducted under anesthesia (40 ppm benzocaine) to minimize animal stress and suffering. Terminal sampling was performed after euthanasia by an overdose of benzocaine (150 ppm).

#### Temperature treatments

The hatched larvae from the male- and female-prone groups were split in two groups at the age of 13 days-post-fertilization (dpf). One group was reared at 16.5°C (low temperature, LT), the control group, and the other group at 20°C (high temperature, HT), the latter to induce environmentally-mediated masculinization. Thus, four combinations were available: male-prone (offspring from sires a and b) at LT, male-prone at HT, female-prone (offspring from sires c and d) at LT and female-prone at HT. Each combination was replicated in two experimental tanks, so that 8 tanks were used during this period (Figure 1A). In each tank, an equal number of albino fish was also included, in order to be able to ascertain that the expected distorted sex ratios at HT were indeed due to the temperature treatments and also to identify any possible tank effect. Temperature treatments lasted until 65 dpf, the end of the thermosensitive period, at which point temperature was raised to 21°C to allow sufficient growth of the LT groups in order to facilitate sexing of the fish at the end of the experiment. From day 135 onwards, temperature was maintained at 20-22°C in all tanks until sampling.

#### Tagging and Samplings

After anesthetic sedation by immersion in 40 ppm of benzocaine, fish were individually tagged with nano-tags (Nonatec) as soon as it was feasible, i.e., when they reached an average weight of ~1.5 g (85 dpf for HT and 105 dpf for LT) to follow their growth rate, which is highly linked to sex determination in the juvenile sea bass 73.

At 323 dpf, fish were sedated in their rearing tank by adding a mild dose of anesthetic, captured and then euthanized with excess anesthesia. A total of 790 experimental and 868 albino fish were measured for body weight and length and were sexed by visual inspection, which allows unambiguous sex identification at this age. Gonad samples were preserved in liquid nitrogen for 200 fish in total: 10 females and 10 males from each one of the four LT tanks (80 fish) and 10 females and 20 males from each one of the four HT tanks (120 fish). The higher number of males was used to take into account for masculinized females in the HT groups. For a summary of the experimental design see Figure 1A.

#### Genotyping

The 790 experimental fish were genotyped for 12 microsatellite markers by Labogena-DNA (Jouy-en-Josas, France). Seven hundred sixty eight of those (97.2%) gave adequate markers' amplification, and 764 (96.7%) were traced back to a single parent pair, using VITASSIGN 74 with 1 mismatch tolerated. It turned out that almost all fish were derived from only one dam, so the remaining fish from the second dam (only 7 in total) were not included in the analyses, which then comprised 755 fish with known pedigree. The number of fish available for analysis of sex ratios from the LT groups was: 42 from sire a, 147 from sire b, 100 from sire c, and 87 from sire d. The number of fish available for analysis from the HT groups was: 59 from sire a, 132 from sire b, 76 from sire c, and 112 from sire d.

**Multiplex bisulfite sequencing (MBS) library preparation and bioinformatics**

Two separate MBS libraries were constructed: the first library (MBS1) contained cyp19a1a amplicons from 183 valid samples (out of 200 original ones) from the offspring gonads as well as the sperm samples of the four sires and the oocytes of the two dams. The second library (MBS2) contained amh-r2, dmrt1, er-β2, foxl2, fsh-r and nr3c1 amplicons in a representative subset of 94 samples and in the 5 parents. An average of 6 individual samples per sire, sex, and temperature treatment was ensured for all groups. For the HT males, the maximum available number of fish were included.

*DNA extraction and bisulfite conversion.* DNA was extracted by the standard phenol-chloroform-isoamyl alcohol (PCI; 25:24:1) protocol. Treatment with 1 μg of proteinase K (Sigma-Aldrich) and 0.5 μg of ribonuclease A (PureLink RNase A; Life Technologies) were used to eliminate the presence of proteins and RNA, respectively. For cryopreserved sperm samples, two PBS washings followed by a 1:5 dilution in PBS preceded the incubation with proteinase K. Five hundred nanograms of DNA per sample were bisulfite converted using the EZ DNA Methylation-Direct™ Kit (Zymo Research; D5023) in two batches of 96-well plates, following the manufacturer's instructions with extended desulphonation time to 30 minutes. Elution of bisulfite converted DNA was performed with 20 μL of Milli-Q autoclaved H2O passing the same volume twice through the column by centrifugation.

*Primers design.* Primers were designed for bisulfite converted DNA using MethPrimer (Table 4).

Table 4. Primers used in MBS library preparation

| Gene symbol | | Primer (5' to 3') | Annealing Tª (°C) |
|---|---|---|---|
| *cyp19a1a* | Forward | TTAGTTTTTCGTTGTTTGTTTTT (SEQ ID NO: 4) | 55 |
| | Reverse | ACCTACAAAAATCATTACCCGTTCA (SEQ ID NO: 5) | |
| *dmrt1* | Forward | TGAGAGTGGGTGTATGTTATTGTTT (SEQ ID NO: 6) | 55 |
| | Reverse | ACTAACAATCCCTCCAATTACAAAA (SEQ ID NO: 7) | |
| *amhr-2* | Forward | GTGGGAAATTTTTTTTATATTTTTAGGA (SEQ ID NO: 8) | 55 |
| | Reverse | ACAACGACCTAAACCCTTTACTACA (SEQ ID NO: 9) | |
| *er-b2* | Forward | ATTATATTTTTATTTTGGTATTTTTTAGTT (SEQ ID NO: 10) | 58 |
| | Reverse | ACCGACATTAAAAATTCCAACTTCCT (SEQ ID NO: 11) | |
| *fshr* | Forward | AATATAGAGGGAAATAATAGTGAGAGAGTG (SEQ ID NO: 12) | 60 |
| | Reverse | AACAAAAACTCAAAATTCGTTTAACCAAAC (SEQ ID NO: 13) | |
| *foxl2* | Forward | TAGTTTGTGAGGATATGTTTGAGAAG (SEQ ID NO: 14) | 55 |
| | Reverse | TTCCCAATAAAAACAATACATCATC (SEQ ID NO: 15) | |
| *nr3c1* | Forward | TTATTGTAGGGATTGGAGGATTAAA (SEQ ID NO: 16) | 55 |
| | Reverse | ACCGCTAACTATCGATCCAATAACA (SEQ ID NO: 17) | |
| Adapters | Forward | TCGTCGGCAGCGTCAGATGTGTATAAGAGACAG (SEQ ID NO:18) | |
| | Reverse | GTCTCGTGGGCTCGGAGATGTGTATAAGAGACAG (SEQ ID NO: 19) | |

Primers were further validated using Primer3Plus (Untergasser A, et al. 2012. Nucleic Acids Res 2012; 40:e115) after in silico bisulfite conversion of the target sequence using Bisulfite Primer Seeker (Zymo Research). Amplicons were designed so that they never exceeded 550 bp in length in order to ascertain the acquisition of overlapping paired-end reads using the 300 bp paired-end Illumina sequencing protocol. An ideal amplicon's range was considered between 450 and 500 bp encompassing as many CpGs as possible. The target regions included as much as possible from the first exon and the promoter, in this order of priority due to the importance of the first exon in the regulation of gene expression 80, of each target gene. At this point it should be recalled that our own recent studies in the European sea bass show that not only the first exon but specially the first intron is very informative of the inverse relationship between DNA methylation and gene expression, so the first intron should be considered in future similar studies. Adapters were added to the 5' ends of the region-specific primers as in Illumina's protocol for 16S metagenomic library preparation. The target region of cyp19a1a included the 7 CpGs studied previously in sea bass 34 at positions 21022651, 21022278, 21022271, 21022255, 21022235, 21022214, and 21022163, encompassing parts of the promoter, 5' UTR and first exon.

*Amplicons PCR.* Amplifications of targeted regions were performed in a total volume of 25 μL containing: 25 ng of DNA (2 μL), 4 mM $MgCl_2$, 0.8 mM dNTPs, primers at 0.8 μM (Life Technologies), 2.5 U of GoTaq G2 Hot Start polymerase (Promega) and its corresponding 5X Green GoTaq Flexi Buffer (Promega). PCR conditions were as follows: 7 minutes at 95°C, followed by 40 cycles of 95°C for 1 minute, annealing temperature between 55 and 60°C depending on each primer pair for 2 minutes and 65°C for 2 minutes, with a final step at 65°C for 10 minutes. The primers were validated by Sanger sequencing of amplicons from a pool of two samples. The presence and size of bands were confirmed by agarose gel electrophoresis in all samples.

*Size-selection and normalization.* After PCR amplification of the target regions, we performed size-selection (MBS1 and MBS2) and normalization of DNA quantities (MBS2) across PCR products following a customized version of the bead-based normalization of Hosomichi et al. (BMC Genomics 2014; 15:645) The working solution of serapure magnetic beads was prepared by washing 2 ml of Sera-mag SpeedBeads (Fisher 09981123) with Tris-EDTA (TE; 10 mM Tris; 1 mM EDTA) and adding the beads in a total volume of 50 mL containing 20% PEG-8000, a concentration of 2.5 M NaCl, 500 mM of Tris-HCl, 1 mM EDTA and 0.00055% Tween 20 (Adapted from: 81). In brief, 8 μl of PCR product and 42 μL of Milli-Q autoclaved H2O were incubated for 5 minutes at room temperature with 20 μL of beads. Following 2 min incubation on the magnetic stand (3D-printed 96-well magnetic rack designed by http://www.thingiverse.com/acadey/ and realized by MAKE Creative Spaces), supernatants were transferred to new wells and incubated for 5 min with 0.8x of magnetic beads for MBS1 and with 0.6x of magnetic beads for MBS2. After discarding the supernatant, a single wash with 70% freshly prepared ethanol was performed and DNA was eluted in 20 μL Milli-Q autoclaved $H_2O$. For MBS2, size-selected PCR products were incubated in equal volumes with 20-fold diluted magnetic beads (PEG 20% and 2.5 M NaCl) and isopropanol. After incubation for 5 minutes at room temperature and washing with 70% freshly prepared ethanol, PCR products were eluted in 20 μL Milli-Q autoclaved $H_2O$. Since each amplicon contained theoretically equal DNA amounts, identical volumes of each amplicon were pooled for each biological sample, resulting in 99 wells containing 6 amplicons each.

*Index PCR and size-selection.* Sample-specific indices were incorporated into the amplicons following a dual-index strategy with i7 indices from Nextera XT index Kit SetA and i5 indices from Nextera XT index Kit SetD (Illumina; FC-131-2001 and FC-131-2004). For MBS1, the Nextera XT index Kit SetA for 94 samples and the Nextera XT index Kit SetD (Illumina; FC-131-2001 and FC-131-2004 accordingly) for 95 samples were used. For MBS2, a combination of indices from the same Nextera XT index kits was used. PCR reactions were performed using the 2x KAPA HiFi HotStart ReadyMix, 5 μL of each primer and 5 μL of template pooled amplicons DNA in a total volume of 50 μL with the following conditions: 95°C for 3 minutes, 8 cycles of 95°C for 30 seconds, 55°C for 30 seconds and 72°C for 30 seconds and a final step at 72°C for 5 minutes, according to Illumina's protocol for 16S metagenomic library preparation. Size-selection and normalization of DNA quantities across samples was carried out after the index PCR according to the customized bead-based normalization using 0.6x of magnetic beads. PCR products were eluted in 15 μL Milli-Q autoclaved $H_2O$ and 2 μL of each sample were pooled together. Therefore, we obtained a single multiplexed library with cyp19a1a for 189 samples and another single multiplexed library with 6 genes (amh-r2, dmrt1, er-β2, foxl2, fsh-r and nr3c1) for 99 samples. After pooling, extra clean-up steps were performed using 0.5x magnetic beads in order to ensure the absence of primers. DNA quantity of final libraries was measured trice by the Qubit dsDNA HS Assay Kit (ThermoFisher Scientific) and the Agilent DNA 1000 chip and DNA High Sensitivity (Agilent) by which the size of bands were also visualized. The multiplexed final libraries were additionally quantified by real-time qPCR using the Kapa system prior to sequencing on a MiSeq (Illumina) using the paired-end 300 bp protocol.

*Multiplex bisulfite sequencing library bioinformatics.* Samples were demultiplexed based on the dual-indices by the instrument's software. Adapters and linker sequences were trimmed for paired-end reads by Trim Galore! (Babraham Bioinformatics), while filtering for low quality bases (Phred score < 20). Quality controls of the data were carried out before and after trimming using FastQC (Babraham Bioinformatics). Trimmed reads were aligned against the in silico bisulfite converted sea bass genome (dicLab1 v1.0c) (Tine M, et al. Nat Commun 2014; 5:5770) using Bismark (v. 0.14.4) (Krueger F, Andrews SR. Bismark: a flexible aligner and methylation caller for Bisulfite-Seq applications. Bioinformatics 2011; 27:1571-2) for both in silico bisulfite conversion and alignments. Mappings were done in three steps in a non-directional way: 1) paired reads were aligned, 2) unmapped reads from the first step were aligned as single reads, and 3) unpaired reads from the first step of trimming were aligned like the unmapped reads. An alignment was considered valid if the score attributed was above f(x) = 0 + -0.6 * read length. The alignments of unmapped (2) and unpaired (3) reads were merged using samtools (v. 1.5) and treated as single-end reads. Alignments were visually inspected using the Integrated Genome Browser 84 and the genomic boundaries and amplicon sizes were confirmed. Methylation calling was performed by the bismark_methylation_extractor of Bismark separately for paired-end and for single-end reads. Paired and single reads were merged for each sample in a single file and the rest of the analysis were carried out using R, Rstudio (R Core Team. R: A Language and Environment for Statistical Computing. Vienna, Austria: R Foundation for Statistical Computing; 2015. Available from: https://www.R-project.org; RStudio Team. RStudio: Integrated Development Environment for R. Boston, MA: RStudio, Inc.; 2015. Available from: http://www.rstudio.com/) and Bioconductor (Huber W, et al. Nat Methods 2015; 12:115-21). Only CpGs with coverage more than 5 were retained for further analysis. For each CpG per sample, counted cytosines and thymines were summed up from paired-end and single-end reads and percent of methylation was calculated as 100*(Cs/Cs+Ts). The bisulfite conversion ratio was calculated as 100 minus the percent of Cs methylated in CHH context from the report of Bismark for paired-end reads. The mean bisulfite conversion ratio was 99.55%.

**General statistical analysis**

All statistical analysis were performed using R (R Core Team 2015; RStudio Team 2015 cited *ad supra*). The tank effect on resulting sex ratios was evaluated by Fisher's exact test for count data. Departures from Fisherian sex ratios and effects of temperature on sex ratios were assessed by Fisher's exact test for count data. The effects of sex, sire and temperature were evaluated on body weight, fork length and condition factor (K), the latter defined as K = 100*(W/L3.02), where W = weight in g and L = length in cm, of the offspring by multifactorial ANOVA for unbalanced data separately. The data were previously tested for homogeneity of variances by Levene's test and the normality of the residuals of the linear regression was assessed by the Shapiro-Wilk normality test using log2-transformed values for body weight and fork length and sine-transformed values for condition factor (K). Methylation data was evaluated by 2-way ANOVAs and the normality of the residuals was tested by the Shapiro-Wilk test. In the cases where the assumption of normality of the residuals was violated, the values were logit-transformed. In the cases where the normality of residuals was violated even with transformed values, 2-way ANOVAs with modified M-estimators and 5000 bootstraps were performed.

**Analysis of DNA methylation data**

*Mean DNA methylation levels.* Association between DNA methylation levels of the CpGs was estimated using Pearson's product-moment correlation coefficients. Mean DNA methylation levels were calculated by averaging the methylation percentages of each CpG per gene per sample and subsequently averaging the overall methylation per grouping factor depending on the comparison. For cyp19a1a, mean methylation levels were calculated using only the 5 central CpGs (21022278, 21022271, 21022255, 21022235 and 21022214) per sample because the two extreme CpGs (positions 21022651 and 21022163) turned out to be always 100% methylated. Two-way ANOVA was used to assess the effects of sex and temperature on mean DNA methylation levels for each gene. The normality of the residuals was tested by the Shapiro-Wilk test. For three genes (er-β2, fsh-r and nr3c1), logit-transformed values were used for the two-way ANOVAs since the assumption of normality of the residuals was otherwise violated. In the case of cyp19a1a and amh-r2, we used two-way ANOVAs with modified M-estimators and 5000 bootstraps (function pbad2way of the WRS2 package v.0.9-2).

*Transmission of DNA methylation patterns.* Next, we focused on individual CpGs, which were 82 in total distributed in 7 genes, in order to investigate, on one hand, the possible transmission of the methylation status from sire to offspring, and, on the other hand, to identify the CpGs that were most responsive to temperature. To study sire-to-offspring transmission, only the offspring reared at low temperature was used to exclude the possible distorting effects of high temperature on methylation levels. For each CpG, the methylation in each offspring was subtracted from the methylation of the corresponding sire and the mean of these differences was calculated per sex and per sire. Thus, positive values indicate hypermethylation in sire and negative values indicate hypermethylation in offspring. The idea was to identify those CpG with the differences, positive or negative, as close as possible to zero. On the other hand, to study the effects of high temperature, for each CpG the mean methylation per sex and sire in offspring reared at low temperature was subtracted from the mean methylation in offspring reared at high temperature. In this case, positive values indicate hypermethylation at high temperature and negative values indicate hypermethylation at low temperature. Here, the idea was to identify those CpGs with the highest differences. The latter approach allowed us to identify the genes that exhibited the most dynamic changes with temperature.

*Epigenetic biomarkers to predict sex.* We used the 26 CpGs of the dmrt1, cyp19a1a and *amhr2* in 93 fish. We filtered the data as follows: 1) we removed 6 samples for which there was no information on the methylation of 15 or more of the CpGs, and 2) we removed 3 CpGs for which there was no information in more than 12 fish. Therefore, for the rest of the analysis we used 23 CpGs in 87 fish. The rest of missing data were imputed by the MICE algorithm that generates multivariate imputations by chained equations using the mice package (v. 2.46.0) (an Buuren S, Groothuis-Oudshoorn K. MICE: Multivariate Imputation by Chained Equations in R. J Stat Softw 2011 [cited 2017 Dec 15], Available from: https://www.jstatsoft.org/article/view/v045i03). We performed Principal Component Analysis (PCA) with 5-fold cross validation. The offspring was divided into 5 groups by the function createFolds of the caret package (v. 6.0-78). Each of the 5 groups was used as test set, the coordinates of which were predicted based on the PCAs performed on the remaining individuals (training sets). In this way, each fish was used as both training and test. The average success of the method was calculated based on the average percent of individuals for which the prediction failed. The results of the PCA and the predicted individuals were visualized using the package factoextra (v. 1.0.5).

II. RESULTS

**Genotyping and sex ratios**

Four European sea bass sires (sires a-d) with different sex tendencies, known from previous experiments to produce more or less females in the offspring, were individually crossed with two dams (Figure 1A). During the thermosensitive period, from 13 to 65 dpf, half of the offspring was reared at low (16.5°C) and the other half at high temperature (21°C) in replicate treatments. From the end of this period and onwards, all fish were kept at 20-22°C until sampling. In total, there were 790 European sea bass sampled at ~11 months available for genotyping and sex ratio analysis. Genotyping unambiguously assigned 764 fish to the parents but showed that most (99%) offspring came from dam b, meaning that the rate of fertilization of the eggs of dam a was low, that the viability of the offspring of dam a was low, or both, something that is frequent in fish. Thus, the few offspring of dam a were no longer considered and only results related to the four families derived from dam b were reported. Considering only the offspring of one dam and increasing sample size allowed us in fact to unambiguously disentangle the sire-specific effects. Sex ratio analysis showed that the offspring of dam b crossed with sires a, b, c or d had different percentages of females: 11.9, 25.2, 35.0 and 48.3%, respectively (Figure 1B). Thus, the progeny of sires a and b, previously shown to give less female offspring, had a lower percent of females than the progeny of sires c and d, previously shown to give more female offspring (Fisher's exact test for count data; p<0.0001). The sex ratios were independent of the replicate tank in which fish were raised and those of the progeny of sires a, b and c significantly departed from the Fisherian sex ratio (Table 5).

Table 5. Fisher's exact test for count data for sex ratios

| | Sire | Odds Ratio | *p*-value |
|---|---|---|---|
| Departure from Fisherian sex ratio | a | 7.214 | 0.001 |
| | b | 2.962 | 1.35g$^{e-05}$ |
| | c | 1.851 | 0.045 |
| | d | 1.071 | 0.880 |
| Effect of temperature | a | 0.263 | 0.124 |
| | b | 0.441 | 0.010 |
| | c | 0.710 | 0.330 |
| | d | 0.394 | 0.003 |

High temperature masculinized a subset of the females (Figure 1B). This masculinization was similar to that observed in the internal control group of albino European sea bass, which were added in each tank (see Materials and Methods) in order to check that the observed effects were indeed due to the temperature treatments and not to spurious tank effects (Figure 2). Masculinization by high temperature was sire-dependent: 72, 49, 21 and 45% of the females were masculinized in the offspring of sires a-d, respectively (percent between the dotted lines inside the LT offspring; Figure 1B) and the effect was significant in the progeny of sires b and d, but not in the progeny of sires a and c (Table S1). Therefore, these results clearly show sire-specific differences not only in sex ratios at control temperature but also in the degree of masculinization as a response to elevated temperature.

As usual in European sea bass, females were bigger than males at the time of sampling, with small differences among the offspring of the different sires. Early exposure to temperature slightly increased growth in both sexes (Figure 3).

**DNA methylation levels of genes related to sexual development in the gametes of the parents and the gonads of the offspring**

The overall DNA methylation profiles of the seven genes related to sexual development were gene-specific and showed a wide range of values (Figure 4). The most extreme values were present in foxl2 (<1%) and er-β2 (>95%). Of the seven CpGs measured in the European sea bass *cyp19a1a* promoter, DNA methylation was essentially 100% in CpGs at positions 21022651 (Figures 5A and 6) and 21022163 (Figures 5B and 6) relative to the transcription start site (TSS) in all fish. Thus, these two CpGs were excluded from further analysis. Among the remaining five CpGs, CpG at position 21022214 showed a strong positive correlation with the rest of the CpGs at positions 21022278, 21022271, 21022255 and 21022235 (ρ=0.77, p<2.2e-16; Figures 5C and 6). Even stronger was the positive correlation of the CpGs at positions 21022278, 21022271, 21022255 and 21022235 (an example of the four possible combinations is shown in Figure 5D and all of them in Figure 6).

In the offspring gonads, and regardless of sire, sex and temperature significantly influenced DNA methylation of cyp19a1a (2-way ANOVA using robust M-estimators and 5000 bootstraps; sex: p=0.027 and temperature: p=0.025). Further, the interaction of both factors was also significant (Sex x Temperature: 0.018). DNA methylation of dmrt1 was also influenced by sex and temperature (2-way ANOVA; sex: p<0.001 and temperature: p=0.016). For cyp19a1a, higher DNA methylation levels were observed in females reared at high temperature, while in dmrt1, the inverse pattern was present, with lower DNA methylation in females reared at high temperature and even lower in males (Figure 4). For the rest of the examined genes (foxl2, amh-r2, er-β2 and fsh-r), non-significant effects of sex and temperature were observed, except from a significant interaction of sex and temperature in nr3c1 (2-way ANOVA; p=0.030; Figure. 4). However, when individual sires were taken into account, with the exception of nr3c1, sire-specific tendencies of increase or decrease of DNA methylation according to sex and/or temperature were evident. For example, in cyp19a1a, in the offspring of sires b and c, there was higher DNA methylation in females reared at high temperature and in males, and the levels of DNA methylation were similar to the ones of the progeny of all sires combined. In the offspring of sire a, however, although the trends were the same, the actual DNA methylation levels were lower in the females reared at low temperature. On the contrary, in sire d, the opposite pattern was evident, with a tendency of lower DNA methylation in females reared at high temperature and in males. These results not only show that there are important differences in absolute methylation levels depending on the gene considered but also the existence of sex-specific differences in methylation levels for some genes, the existence of gene- and sex-specific responses to temperature and, finally, that there also are sire-specific differences. Together, they clearly illustrate that both the genetic and environmental contribution to DNA methylation of specific genes are important for sexual development in this fish.

We also measured DNA methylation in the oocytes of dam b, the one from which all analyzed offspring were derived, as well as the sperm of the four sires. For five out of the seven analyzed genes (cyp19a1a, foxl2, amh-r2, er-β2 and fsh-r), DNA methylation in oocytes was lower than in sperm. In the case of dmrt1 it was the other way round: methylation in the oocytes was higher than in the sperm, whereas nr3c1 showed very stable methylation levels across parents, resembling the situation described above concerning the offspring. Focusing on the sperm, sire a departed from the rest in the sense that DNA methylation of some genes (cyp19a1a, dmrt1, fsh-r) was clearly lower when compared to values found in the other sires; for foxl2 sire a, in contrast, exhibited the highest DNA methylation levels. These results also show clear gene- and sire-specific differences in methylation but, in general, methylation in sperm was higher than in oocytes, although this should be taken with caution since only one dam was analyzed.

**Stable and dynamic CpGs from parents to offspring**

Next, to identify stable methylation patterns from parents to offspring, it was calculated, for each gene, the methylation difference between levels in parents (specifically, the sperm of the different sires) minus levels in offspring for each analyzed CpG. To do this, both sexes were contemplated but only fish reared at low temperature were used to exclude possible environmental influences. Methylation differences close to zero were considered indicative of stable methylation levels, whereas differences above and below zero indicated hypomethylation and hypermethylation, respectively, in offspring vs. parents. Methylation differences depended on individual CpGs within a given gene, the sex and the sire (Figure 7A). However, regardless of sex and sire, the more stable levels of methylation were observed in the CpGs of er-β2, foxl2 and nr3c1 (Figure 7B). These low differences were independent from the actual methylation levels in the sires, whether close to 0 or close to 100%, although there were no intermediate values to compare, suggesting that low difference values had not their origin in low or very low methylation levels. Thus, genes were grouped into two categories, stable (er-β2, foxl2 and nr3c1) or dynamic (cyp19a1a, dmrt1, fshr and amh-r2) independently of sex (Figure 8).

The same approach based on the analysis of differences was used to identify the CpGs most responsive to temperature. In this case, the CpGs with the highest methylation differences between fish exposed to HT vs LT was searched for. Again, changes were both gene- and sire-dependent (Figure 9A). Nevertheless, and regardless of sire and even sex, the methylation of CpGs of dmrt1 and cyp19a1a showed the biggest differences between offspring exposed to high and low temperature. Additionally, the CpGs of amh-r2 were responsive to temperature, but only in male offspring (Figure 9B). As before, these temperature-dependent differences in methylation in specific CpGs were independent of the actual methylation levels (Figure 10).

**Prediction of phenotypic sex by analysis of epigenetic profiles: epigenetic biomarkers**

Based on the above, it was hypothesized that the methylation of the CpGs of the genes most influenced by temperature in this species with mixed genetic and environmental sex determination might be indicative of the sex. Therefore, we performed Principal Component Analysis (PCA) with 5-fold cross validation with 87 fish which represented the offspring for which information on all these genes was available, using the CpGs (n=23) of these genes as variables. The five independent validations gave similar results and one of them is shown as representative (Figure 11A). The first two dimensions of the PCA explained 61.9% of the total variation and the 72 individuals of the training set were clearly separated according to their sex (blue and red dots, Figure 11A). For the remaining 15 individuals (the test set), the sex was predicted using the information of the training set (Figure 11A). Only for two male individuals (arrows, Figure 11A) out of 15 in total, the sex was incorrectly predicted. The average success rate for this method of predicting sex, using 5-fold cross validation, based only on methylation information of the CpG of these genes was 88.09%. Up to date, this constitutes the first prediction of phenotypic sex using methylation status in an animal.

**Example 2**

**Prediction of phenotypic sex by analysis of epigenetic profiles: epigenetic biomarkers**

I. MATERIAL AND METHODS

**Dissection of tissues**

Dissect tissues from the fish with proper material. In the development of the method, gonads of juvenile fish were used, but gonads of older or younger fish can also be used. Also, tissue fragments where gonads are contained, for example, trunk sections, would also work. Dissected tissues can be processed immediately or stored frozen until analysis.

**Extraction of genomic DNA**

Extract genomic DNA by any conventional method for DNA extraction. In the development of the method, the standard phenol-chloroform-isoamyl alcohol (PCI; 25:24:1) protocol was used. Treatment with 1 μg of proteinase K and 0.5 μg of ribonuclease A were used to eliminate the presence of proteins and RNA, respectively.

**Preparation of genomic DNA for DNA methylation analysis**

The method proper starts here once genomic DNA has been obtained from each sample (1 sample = 1 fish).

In the development of the method, the standard bisulfite conversion was used but other methods available that allow quantification of methylation levels at single nucleotide resolution would also work. In the development of the method, 500 ng of DNA per sample were bisulfite converted using the EZ DNA Methylation-Direct™ Kit (Zymo Research; D5023) in two batches of 96-well plates, following the manufacturer's instructions with extended desulphonation time to 30 minutes. Elution of bisulfite converted DNA was performed with 20 μl of Milli-Q autoclaved H2O passing the same volume twice through the column by centrifugation.

**Obtention of information on specific CpG in two genes: cyp19a1a and dmrt1**

The method has a whole has three key aspects. Knowing what genes to focus on is the first key aspect.

In the development of the method, PCR amplification of two genomic regions corresponding to cyp19a1a and dmrt1 genes was used but other methods that allow obtaining information on the genomic regions of these genes such as, for example, Whole Genome Bisulfite sequencing, can be used.

PCR amplifications of targeted regions were performed in a total volume of 25 μL containing: 25 ng of DNA (2 μL), 4 mM $MgCl_2$, 0.8 mM dNTPs, primers at 0.8 μM (Life Technologies), 2.5 U of GoTaq G2 Hot Start polymerase (Promega) and its corresponding 5X Green GoTaq Flexi Buffer (Promega). PCR conditions were as follows: 7 minutes at 95°C, followed by 40 cycles of 95°C for 1 minute, annealing temperature 55°C for 2 minutes and 65°C for 2 minutes, with a final step at 65°C for 10 minutes. The primers used are shown in Table 6 below.

Table 6. Primer pairs used for amplifying the *cyp19a1a* gene and *dmrt1* gene.

| Gene symbol | | Primer (5' to 3') | Genomic position in the European sea bass genome (Chromosome: position) |
|---|---|---|---|
| *cyp19a1a* | Forward | TTAGTTTTTCGTTGTTTGTTTTT (SEQ ID NO: 4) | LG5: 21022143 - 21022665 |
| | Reverse | ACCTACAAAAATCATTACCCGTTCA (SEQ ID NO: 5) | |

(continued)

| Gene symbol | | Primer (5' to 3') | Genomic position in the European sea bass genome (Chromosome: position) |
|---|---|---|---|
| *dmrt1* | Forward | TGAGAGTGGGTGTATGTTATTGTTT (SEQ ID NO: 6) | LG20: 18505581 - 18506025 |
| | Reverse | ACTAACAATCCCTCCAATTACAAAA (SEQ ID NO: 7) | |

**Obtention of information at single nucleotide resolution of the specific cytosine-guanine dinucleotides (CpGs).**

In the development of the method, adapters with specific indices were attached to each sample, the samples were multiplexed in a final library and sequenced on an Illumina MiSeq instrument. Other ways to sequence, such as Sanger or Nanopore would also work. For the prediction of sex in a single sample, no indexing and multiplexing is needed. For more than one sample, other ways of distinction between samples may be used, for example, sequencing in individual lanes.

**Extraction of methylation values corresponding to the following specific CpGs.**

Knowing the genomic location of the following 19 specific CpGs positions is the second key aspect of the method.

Table 7. CpG sites location in the European sea bass genome. The genomic coordinates are calculated based on the version 1 (v1.0c) of the European sea bass genome (dicLab1), as annotated by the official public site of access to the genome (http://seabass.mpipz.de/) and accessed in August 2018.

| Gene name | CpG site | CpG (Chromosome:position) European sea bass genome |
|---|---|---|
| Doublesex- and mab-3-related transcription factor 1<br><br>Gene symbol: *dmrt1* | Position 46 of SEQ ID NO: 1 | LG20:18505626 |
| | Position 57 of SEQ ID NO: 1 | LG20:18505637 |
| | Position 74 of SEQ ID NO: 1 | LG20:18505680 |
| | Position 108 of SEQ ID NO: 1 | LG20:18505688 |
| | Position 118 of SEQ ID NO: 1 | LG20:18505698 |
| | Position 152 of SEQ ID NO: 1 | LG20:18505732 |
| | Position 167 of SEQ ID NO: 1 | LG20:18505747 |
| | Position 211 of SEQ ID NO: 1 | LG20:18505791 |
| | Position 217 of SEQ ID NO: 1 | LG20:18505797 |
| | Position 310 of SEQ ID NO: 1 | LG20:18505890 |
| | Position 324 of SEQ ID NO: 1 | LG20:18505904 |
| | Position 365 of SEQ ID NO: 1 | LG20:18505945 |
| | Position 379 of SEQ ID NO: 1 | LG20:18505959 |
| | Position 410 of SEQ ID NO: 1 | LG20:18505990 |
| Cytochrome P450 19A1a (Aromatase)<br><br>Gene symbol: *cyp19a1a* | Position 388 of SEQ ID NO: 2 | LG5:21022278 |
| | Position 395 of SEQ ID NO: 2 | LG5:21022271 |
| | Position 411 of SEQ ID NO: 2 | LG5:21022255 |
| | Position 431 of SEQ ID NO: 2 | LG5:21022235 |
| | Position 452 of SEQ ID NO: 2 | LG5:21022214 |

**Estimation on sex**

1.- For each sample, enter the percent methylation for each one of the 19 CpG positions in the right column in the table below (Table 8)

Table 8. Percent methylation for each one of the CpG positions

| CpG position | Principal component coefficient a ($a_i$ in formula) | Principal component coefficient b ($b_i$ in formula) | Mean reference value (m in formula) | Standard deviation reference value ($d_i$ in formula) | Percent (0-100%) methylation ($X_i$ in the formula) |
|---|---|---|---|---|---|
| | **19 CpGs** | | | | |
| Position 46 of SEQ ID NO: 1 | 0.236 | -0.034 | 6.767 | 4.872 | |
| Position 57 of SEQ ID NO: 1 | 0.199 | -0.052 | 1.704 | 2.093 | |
| Position 74 of SEQ ID NO: 1 | 0.232 | -0.040 | 4.955 | 3.946 | |
| Position 108 of SEQ ID NO: 1 | 0.238 | -0.057 | 9.917 | 8.530 | |
| Position 118 of SEQ ID NO: 1 | 0.184 | -0.014 | 4.952 | 4.276 | |
| Position 152 of SEQ ID NO: 1 | 0.263 | -0.051 | 12.589 | 8.725 | |
| Position 167 of SEQ ID NO: 1 | 0.298 | -0.068 | 17.143 | 10.548 | |
| Position 211 of SEQ ID NO: 1 | 0.261 | -0.055 | 8.141 | 6.390 | |
| Position 217 of SEQ ID NO: 1 | 0.269 | -0.069 | 9.657 | 6.568 | |
| Position 310 of SEQ ID NO: 1 | 0.300 | -0.042 | 24.680 | 15.507 | |
| Position 324 of SEQ ID NO: 1 | 0.294 | -0.028 | 29.674 | 16.929 | |

(continued)

| CpG position | Principal component coefficient a ($a_i$ in formula) | Principal component coefficient b ($b_i$ in formula) | Mean reference value (m in formula) | Standard deviation reference value ($d_i$ in formula) | Percent (0-100%) methylation ($X_i$ in the formula) |
|---|---|---|---|---|---|
| | **19 CpGs** | | | | |
| Position 365 of SEQ ID NO: 1 | 0.286 | -0.045 | 21.450 | 12.053 | |
| Position 379 of SEQ ID NO: 1 | 0.298 | -0.015 | 38.465 | 22.032 | |
| Position 410 of SEQ ID NO: 1 | 0.300 | -0.016 | 24.491 | 13.934 | |
| Position 388 of SEQ ID NO: 2 | -0.046 | -0.455 | 60.217 | 23.261 | |
| Position 395 of SEQ ID NO: 2 | -0.067 | -0.450 | 55.234 | 20.879 | |
| Position 411 of SEQ ID NO: 2 | -0.083 | -0.450 | 66.392 | 25.016 | |
| Position 431 of SEQ ID NO: 2 | -0.090 | -0.443 | 68.647 | 25.113 | |
| Position 452 of SEQ ID NO: 2 | -0.063 | -0.404 | 77.204 | 19.762 | |
| | **20 CpGs** | | | | |
| Position 46 of SEQ ID NO: 1 | 0.236 | -0.033 | 6.767 | 4.872 | |
| Position 57 of SEQ ID NO: 1 | 0.199 | -0.052 | 1.704 | 2.093 | |
| Position 74 of SEQ ID NO: 1 | 0.232 | -0.041 | 4.955 | 3.946 | |

(continued)

| | 20 CpGs | | | | |
|---|---|---|---|---|---|
| Position 108 of SEQ ID NO: 1 | 0.238 | -0.056 | 9.917 | 8.530 | |
| Position 118 of SEQ ID NO: 1 | 0.184 | -0.013 | 4.952 | 4.276 | |
| Position 152 of SEQ ID NO: 1 | 0.262 | -0.053 | 12.589 | 8.725 | |
| Position 167 of SEQ ID NO: 1 | 0.298 | -0.069 | 17.143 | 10.548 | |
| Position 211 of SEQ ID NO: 1 | 0.261 | -0.056 | 8.141 | 6.390 | |
| Position 217 of SEQ ID NO: 1 | 0.269 | -0.070 | 9.657 | 6.568 | |
| Position 310 of SEQ ID NO: 1 | 0.300 | -0.042 | 24.680 | 15.507 | |
| Position 324 of SEQ ID NO: 1 | 0.293 | -0.029 | 29.674 | 16.929 | |
| Position 365 of SEQ ID NO: 1 | 0.286 | -0.045 | 21.450 | 12.053 | |
| Position 379 of SEQ ID NO: 1 | 0.299 | -0.015 | 38.465 | 22.032 | |
| Position 410 of SEQ ID NO: 1 | 0.299 | -0.018 | 24.491 | 13.934 | |
| Position 15 of SEQ ID NO: 2 | 0.019 | 0.046 | 99.536 | 0.430 | |

(continued)

| | | | | | |
|---|---|---|---|---|---|
| | 20 CpGs | | | | |
| Position 388 of SEQ ID NO: 2 | -0.046 | -0.454 | 60.217 | 23.261 | |
| Position 395 of SEQ ID NO: 2 | -0.067 | -0.449 | 55.234 | 20.879 | |
| Position 411 of SEQ ID NO: 2 | -0.083 | -0.449 | 66.392 | 25.016 | |
| Position 431 of SEQ ID NO: 2 | -0.090 | -0.442 | 68.647 | 25.113 | |
| Position 452 of SEQ ID NO: 2 | -0.063 | -0.404 | 77.204 | 19.762 | |
| | 23 CpGs | | | | |
| Position 208 of SEQ ID NO: 3 | 0.019 | -0.205 | 37.208 | 7.320 | |
| Position 293 of SEQ ID NO: 3 | 0.028 | -0.159 | 60.158 | 8.477 | |
| Position 94 of SEQ ID NO: 3 | -0.037 | 0.088 | 99.239 | 1.509 | |
| Position 46 of SEQ ID NO: 1 | -0.235 | -0.042 | 6.767 | 4.872 | |
| Position 57 of SEQ ID NO: 1 | -0.199 | -0.050 | 1.704 | 2.093 | |
| Position 74 of SEQ ID NO: 1 | -0.231 | -0.051 | 4.955 | 3.946 | |
| Position 108 of SEQ ID NO: 1 | -0.238 | -0.057 | 9.917 | 8.530 | |

(continued)

| | 23 CpGs | | | | |
|---|---|---|---|---|---|
| Position 118 of SEQ ID NO: 1 | -0.184 | -0.007 | 4.952 | 4.276 | |
| Position 152 of SEQ ID NO: 1 | -0.261 | -0.066 | 12.589 | 8.725 | |
| Position 167 of SEQ ID NO: 1 | -0.296 | -0.078 | 17.143 | 10.548 | |
| Position 211 of SEQ ID NO: 1 | -0.260 | -0.057 | 8.141 | 6.390 | |
| Position 217 of SEQ ID NO: 1 | -0.268 | -0.080 | 9.657 | 6.568 | |
| Position 310 of SEQ ID NO: 1 | -0.299 | -0.041 | 24.680 | 15.507 | |
| Position 324 of SEQ ID NO: 1 | -0.293 | -0.026 | 29.674 | 16.929 | |
| Position 365 of SEQ ID NO: 1 | -0.285 | -0.044 | 21.450 | 12.053 | |
| Position 379 of SEQ ID NO: 1 | -0.298 | -0.012 | 38.465 | 22.032 | |
| Position 410 of SEQ ID NO: 1 | -0.298 | -0.026 | 24.491 | 13.934 | |
| Position 15 of SEQ ID NO: 2 | -0.019 | 0.047 | 99.536 | 0.430 | |
| Position 388 of SEQ ID NO: 2 | 0.050 | -0.438 | 60.217 | 23.261 | |

(continued)

| | 23 CpGs | | | | |
|---|---|---|---|---|---|
| Position 395 of SEQ ID NO: 2 | 0.071 | -0.432 | 55.234 | 20.879 | |
| Position 411 of SEQ ID NO: 2 | 0.087 | -0.430 | 66.392 | 25.016 | |
| Position 431 of SEQ ID NO: 2 | 0.094 | -0.419 | 68.647 | 25.113 | |
| Position 452 of SEQ ID NO: 2 | 0.066 | -0.385 | 77.204 | 19.762 | |

2.- For each sample, calculate Y1 and Y2 using the formulas below. If using 19 CpGs:

$$Y1 = \sum_{i=1}^{19} a_i \frac{(X_i - m_i)}{d_i}$$

$$Y2 = \sum_{i=1}^{19} b_i \frac{(X_i - m_i)}{d_i}$$

If using 20 CpGs:

$$Y1 = \sum_{i=1}^{20} a_i \frac{(X_i - m_i)}{d_i}$$

$$Y2 = \sum_{i=1}^{20} b_i \frac{(X_i - m_i)}{d_i}$$

If using 23 CpGs:

$$Y1 = \sum_{i=1}^{23} a_i \frac{(X_i - m_i)}{d_i}$$

$$Y2 = \sum_{i=1}^{23} b_i \frac{(X_i - m_i)}{d_i}$$

where each element in the formula is defined in the table headings above.

3.- For each sample, calculate the female probability, $F^p = \frac{1}{1+e^{-p}}$, where If using 19 CpGs:

$$P = 0.4186 - 0.7749\,Y1 - 0.1297\,Y2$$

If using 20 CpGs:

$$P = 0.4176 - 0.7755\,Y1 - 0.1298\,Y2$$

If using 23 CpGs:

$$P = 0.4397 + 0.7869\,Y1 - 0.1672\,Y2$$

4. Calculate $F^p = \frac{1}{1+e^{-p}}$,
5. Determine sex:

If $F^p > 0.5$ the fish is a female
If $F^p < 0.5$ the fish is a male

II. RESULTS

The overall relationship between dmrt1 and cyp19a1a of representative values at each CpG are in Figure 12 for males and females, although absolute values can vary depending on biological samples of origin.

If a given sample corresponds, for example, to a female fish, following the procedure described above in the section "Prediction of phenotypic sex by analysis of epigenetic profiles: epigenetic biomarkers" will result in a plot like the one shown in Figure 13 because the $F^p > 0.5$.

The method has an estimated accuracy of 88%, i.e., essentially it correctly identifies the sex of almost 9 of each 10 samples.

## Claims

**1.** An *in vitro* method for predicting the sex in fish comprising

(a) Determining the methylation levels at the following 19 CpG sites of

(i) the *dmrt1* gene promoter within the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, wherein the CpG sites of dmrt1 gene promoter are at position 18,505,626, at position 18,505,637, at position 18,505,680, at position 18,505,688, at position 18,505,698, at position 18,505,732, at position 18,505,747, at position 18,505,791, at position 18,505,797, at position 18,505,890, at position 18,505,904, at position 18,505,945, at position 18,505,959, and at position 18,505,990, in a biological sample isolated from the fish, and
(ii) the *cyp19a1a* gene promoter within the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome, wherein the CpG sites of cyp19a1a gene promoter are at position 21,022,278, at position 21,022,271, at position 21,022,255, at position 21,022,235, and at position 21,022,214, and

(b) using the methylation levels determined in step (a) to obtain the probability of being a female, $F^P$, ($F^P = \frac{1}{1+e^{-P}}$) where

$$P = 0.4186 - 0.7749\,Y1 - 0.1297\,Y2$$

where

$$Y1 = \sum_{i=1}^{19} a_i \frac{(X_i - m_i)}{d_i}$$

and

$$Y2 = \sum_{i=1}^{19} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i.
wherein

- if $F^p > 0.5$ the fish is a female
- if $F^p < 0.5$ the fish is a male

**2.** Method according to claim 1, wherein step (a) further comprises determining the methylation levels of the CpG site of *cyp19a1a* gene promoter at position 21,022,651 of the chromosome 5 of the European seabass genome, and step b) comprises using the methylation levels determined in step (a) to obtain the probability of being a female, $F^P$,

($F^p = \frac{1}{1+e^{-P}}$) where P = 0.4176 - 0.7755 Y1 - 0.1298 Y2

where

$$Y1 = \sum_{i=1}^{20} a_i \frac{(X_i - m_i)}{d_i}$$

and

$$Y2 = \sum_{i=1}^{20} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i,
wherein

- if $F^p > 0.5$ the fish is a female
- if $F^p < 0.5$ the fish is a male.

**3.** Method according to claim 1 or 2, wherein step (a) further comprises determining the methylation levels of at CpG sites of amh-r2 gene promoter within the DNA region comprising a nucleotide sequence having a sequence identity of at least 90% to the DNA region from nucleotide 20275417 to nucleotide 20275717 (SEQ ID NO: 3) of the chromosome LG1A of the European seabass genome, wherein

- the CpG sites of amh-r2 gene promoter are at position 20275424, at position 20275509 and at position 2027562, and

- using the methylation levels determined in step (a) to obtain the probability of being a female, $F^P$, ($F^p = \frac{1}{1+e^{-P}}$) where

$$P = = 0.4397 + 0.7869\,Y1 - 0.1672\,Y2$$

where

$$Y1 = \sum_{i=1}^{23} a_i \frac{(X_i - m_i)}{d_i}$$

and

$$Y2 = \sum_{i=1}^{23} b_i \frac{(X_i - m_i)}{d_i}$$

and where $a_i$ is the Principal component coefficient a for position i, $b_i$ is the Principal component coefficient b for position i, $m_i$ is the Mean reference value for position i, $d_i$ is the Standard deviation reference value for position i, and $X_i$ is the percent (0-100%) methylation value of the sample for position i.
wherein

- if $F^p > 0.5$ the fish is a female
- if $F^p < 0.5$ the fish is a male

**4.** Method according to any one of claims 1 to 3, wherein the biological sample is from the gonads of the fish.

**5.** Method according to any one of claims 1 to 4, wherein the fish is a bass, preferably, the fish is a European sea bass (*Dicentrarchus labrax*), spotted European sea bass (*Dicentrarchus punctatus*), striped bass (*Morone saxatilis*), white bass (*Morone chrysops*), largemouth bass (*Micropterus salmoides*), redeye bass (*Micropterus coosae*), spotted bass (*Micropterus punctulatus*), shoal bass (*Micropterus cataractae*), smallmouth bass (*Micropterus dolomieu*), choctaw bass (*Micropterus haiaka*), Japanese seabass (*Lateolabrax japonicus*), or blackfin sea bass (*Lateolabrax latus*).

**6.** Method according to any one of claims 1 to 5, wherein the step (a) includes the steps of:

(i) treating genomic DNA obtained from a sample with the compound modifying an unmethylated cytosine base or the methylation sensitive restriction enzyme; and
(ii) measuring the methylation level of the treated DNA by one or more methods selected from the group consisting of methylation-specific polymerase chain reaction, real time methylation-specific polymerase chain reaction, PCR using a methylated DNA-specific binding protein, quantitative PCR, pyrosequencing and bisulfite sequencing using primers capable of amplifying the methylated region at the CpG site of the gene.

**7.** Method of claim 6, wherein the compound modifying an unmethylated cytosine base is bisulfite or a salt thereof.

**8.** Method according to claim 6 or 7, wherein the polymerase chain reaction comprises the pair of primers SEQ ID NO: 4 and SEQ ID NO: 5, and/or SEQ ID NO: 6 and SEQ ID NO: 7.

**9.** Use of the methylation levels at the CpG sites of the *dmrt1* gene promoter and of the *cyp19a1a* gene promoter for determining the sex of fish, wherein

- the CpG sites of *dmrt1* gene promoter are at position 18,505,626, at position 18,505,637, at position 18,505,680, at position 18,505,688, at position 18,505,698, at position 18,505,732, at position 18,505,747, at position 18,505,791, at position 18,505,797, at position 18,505,890, at position 18,505,904, at position 18,505,945, at position 18,505,959, and at position 18,505,990 from the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, and
- the CpG sites of *cyp19a1a* gene promoter are at position 21,022,278, at position 21,022,271, at position 21,022,255, at position 21,022,235, and at position 21,022,214 from the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome.

**10.** Use according to claim 9, further comprising:

- the methylation levels of the CpG site of *cyp19a1a* gene promoter at position 21,022,651 of the chromosome 5 of the European seabass genome, and/or
- the methylation levels of the CpG sites of amh-r2 gene promoter at position 20275424, at position 20275509 and/or at position 20275623 from the DNA region comprising a nucleotide sequence having a sequence identity of at least 90% to the DNA region from 20275417 to nucleotide 20275717 (SEQ ID NO: 3) of the chromosome LG1A of the European seabass genome.

**11.** A kit comprising nucleic acid primers specifically designed to hybridize with the DNA region comprising a nucleotide sequence having a sequence identity of at least 90% to the DNA region from nucleotide 18,505,581 to nucleotide 18,506,025 (SEQ ID NO: 1) of the chromosome 20 of the European seabass genome, and nucleic acid primers specifically designed to hybridize with the DNA region comprising a nucleotide sequence having a sequence identity of at least 90% to the DNA region from nucleotide 21,022,143 to nucleotide 21,022,665 (SEQ ID NO: 2) of the chromosome 5 of the European seabass genome.

**12.** Kit according to claim 11, further comprising nucleic acid primers specifically designed to hybridize with the DNA region comprising a nucleotide sequence having a sequence identity of at least 90% to the DNA region from nucleotide 20275417 to nucleotide 20275717 (SEQ ID NO: 3) of the chromosome LG1A of the European seabass genome.

**13.** Kit according to claim 11 or 12, wherein the polymerase chain reaction comprises the pair of primers SEQ ID NO: 4 and SEQ ID NO: 5, and/or SEQ ID NO: 6 and SEQ ID NO: 7.

**14.** Use of a kit according to any one of claims 13 to 13 for determining the sex of fish, preferably, the fish is a bass, more preferably, the bass is a European sea bass (*Dicentrarchus labrax*), spotted European sea bass (*Dicentrarchus punctatus*), striped bass (*Morone saxatilis*), white bass (*Morone chrysops*), largemouth bass (*Micropterus salmoides*), redeye bass (*Micropterus coosae*), spotted bass (*Micropterus punctulatus*), shoal bass (*Micropterus cataractae*), smallmouth bass (*Micropterus dolomieu*), choctaw bass (*Micropterus haiaka*), Japanese seabass (*Lateolabrax japonicus*), or blackfin sea bass (*Lateolabrax latus*).

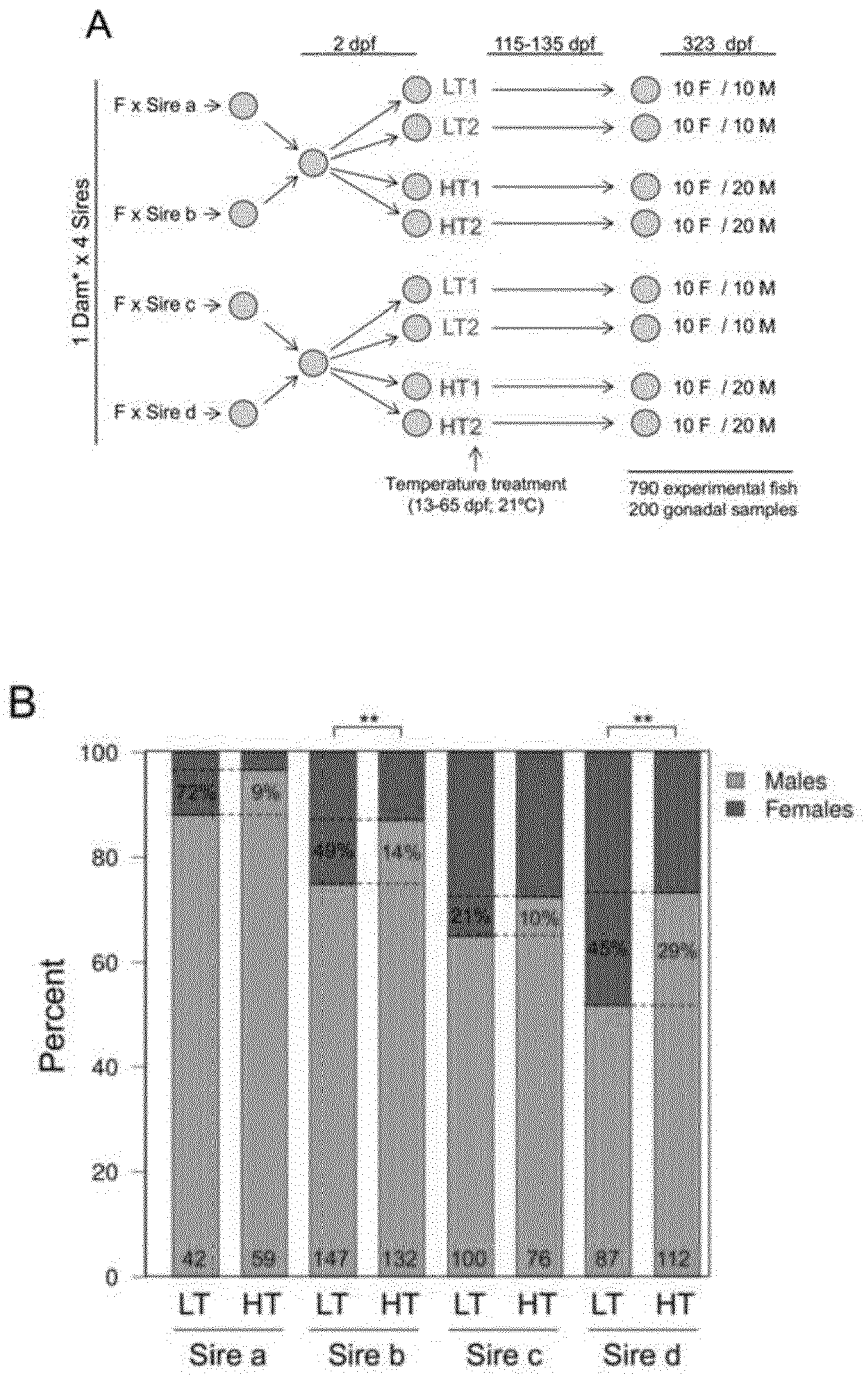
A
2 dpf
115-135 dpf
323 dpf
1 Dam* x 4 Sires
F x Sire a
F x Sire b
F x Sire c
F x Sire d
LT1
LT2
HT1
HT2
10 F / 10 M
10 F / 20 M
Temperature treatment
(13-65 dpf; 21°C)
790 experimental fish
200 gonadal samples
B
Percent
100
80
60
40
20
0
Males
Females
**
72%
9%
49%
14%
21%
10%
45%
29%
42
59
147
132
100
76
87
112
LT
HT
Sire a
Sire b
Sire c
Sire d

FIG. 1

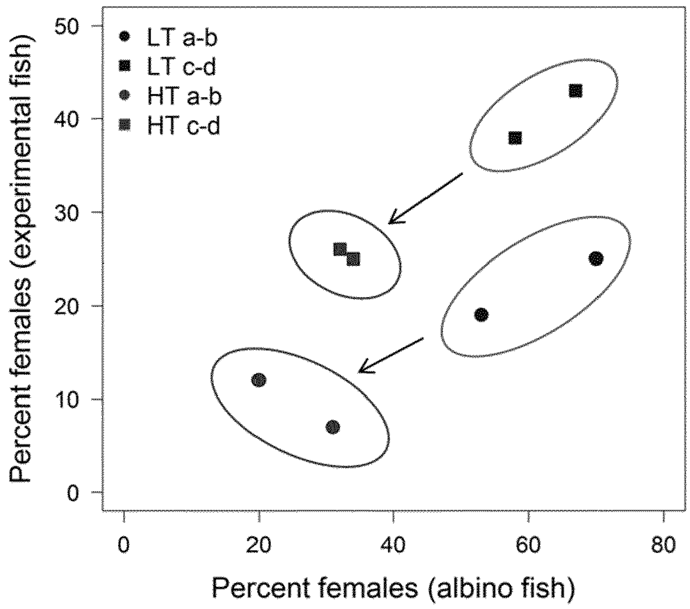
LT a-b
LT c-d
HT a-b
HT c-d
Percent females (experimental fish)
Percent females (albino fish)
0
10
20
30
40
50
0
20
40
60
80

FIG. 2

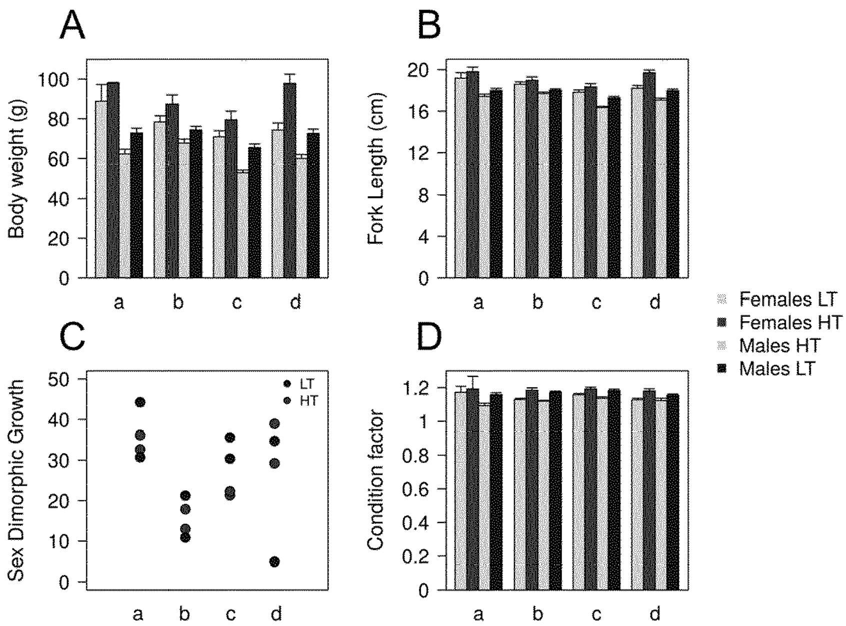
A
B
C
D
Body weight (g)
Fork Length (cm)
Sex Dimorphic Growth
Condition factor
a
b
c
d
LT
HT
Females LT
Females HT
Males HT
Males LT

FIG: 3

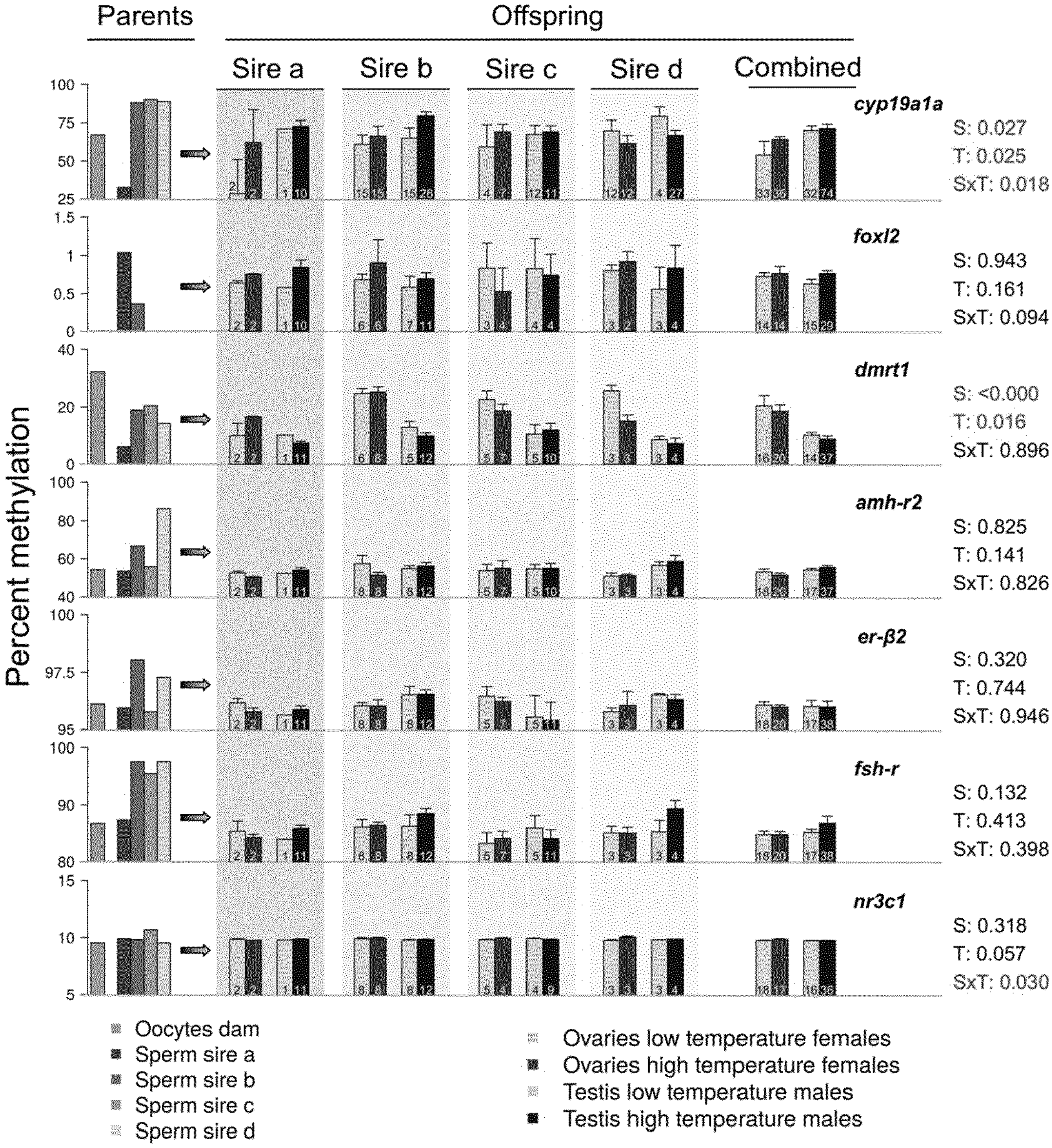

Parents
Offspring
Sire a
Sire b
Sire c
Sire d
Combined
Percent methylation
cyp19a1a
S: 0.027
T: 0.025
SxT: 0.018
foxl2
S: 0.943
T: 0.161
SxT: 0.094
dmrt1
S: <0.000
T: 0.016
SxT: 0.896
amh-r2
S: 0.825
T: 0.141
SxT: 0.826
er-β2
S: 0.320
T: 0.744
SxT: 0.946
fsh-r
S: 0.132
T: 0.413
SxT: 0.398
nr3c1
S: 0.318
T: 0.057
SxT: 0.030
Oocytes dam
Sperm sire a
Sperm sire b
Sperm sire c
Sperm sire d
Ovaries low temperature females
Ovaries high temperature females
Testis low temperature males
Testis high temperature males


FIG. 4

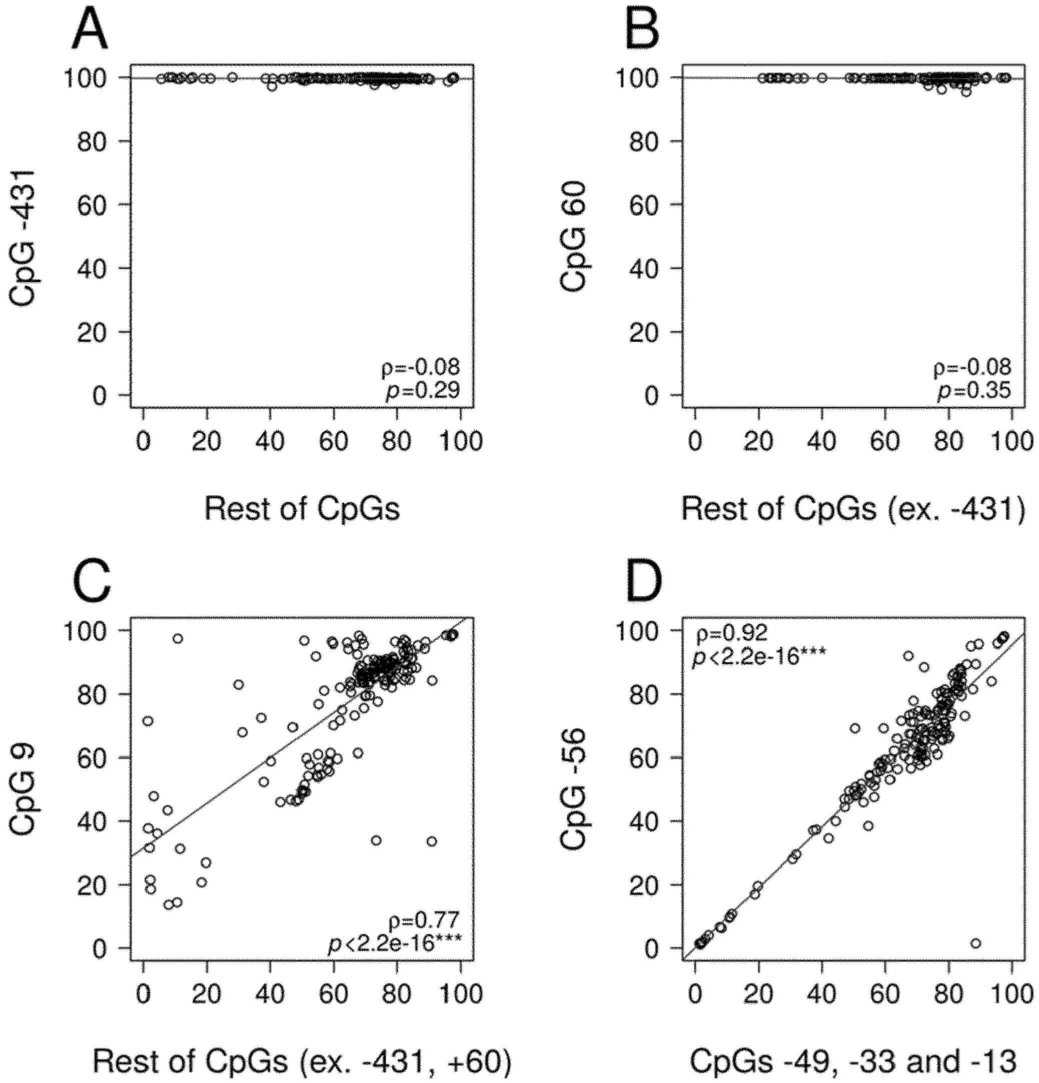

A
CpG -431
ρ=-0.08
p=0.29
Rest of CpGs
B
CpG 60
ρ=-0.08
p=0.35
Rest of CpGs (ex. -431)
C
CpG 9
ρ=0.77
p<2.2e-16***
Rest of CpGs (ex. -431, +60)
D
CpG -56
ρ=0.92
p<2.2e-16***
CpGs -49, -33 and -13


FIG. 5

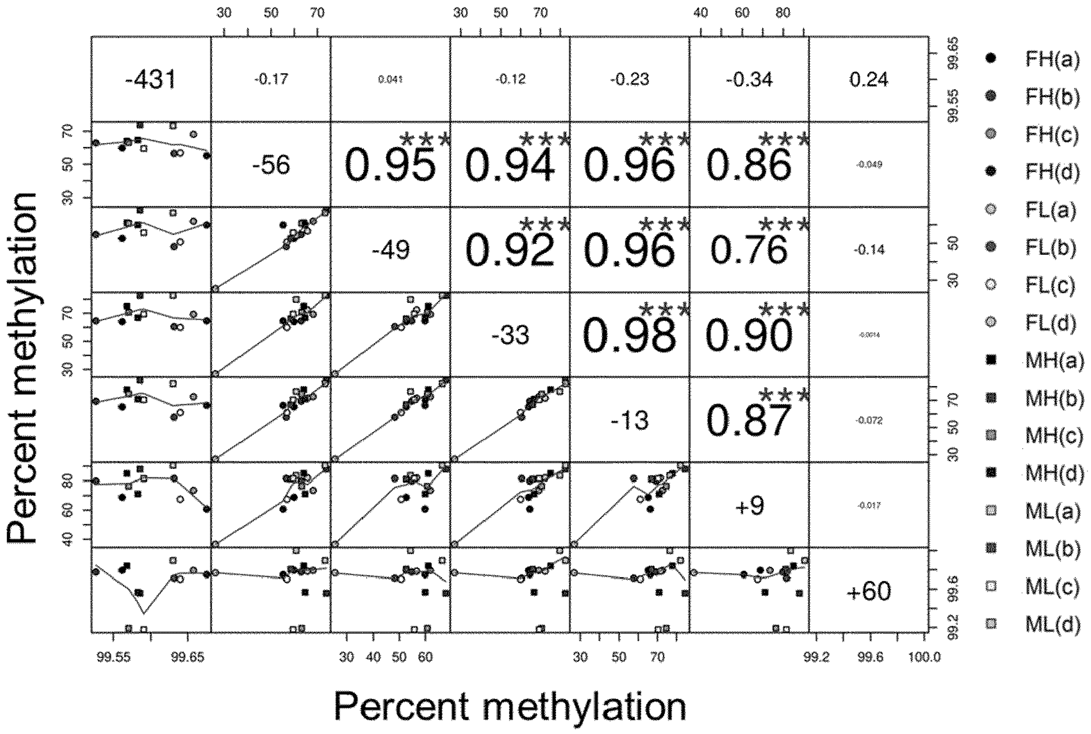
-431
-0.17
0.041
-0.12
-0.23
-0.34
0.24
-56
0.95 ***
0.94 ***
0.96 ***
0.86 ***
-0.049
-49
0.92 ***
0.96 ***
0.76 ***
-0.14
-33
0.98 ***
0.90 ***
-0.0014
-13
0.87 ***
-0.072
+9
-0.017
+60
FH(a)
FH(b)
FH(c)
FH(d)
FL(a)
FL(b)
FL(c)
FL(d)
MH(a)
MH(b)
MH(c)
MH(d)
ML(a)
ML(b)
ML(c)
ML(d)
Percent methylation
Percent methylation

FIG. 6

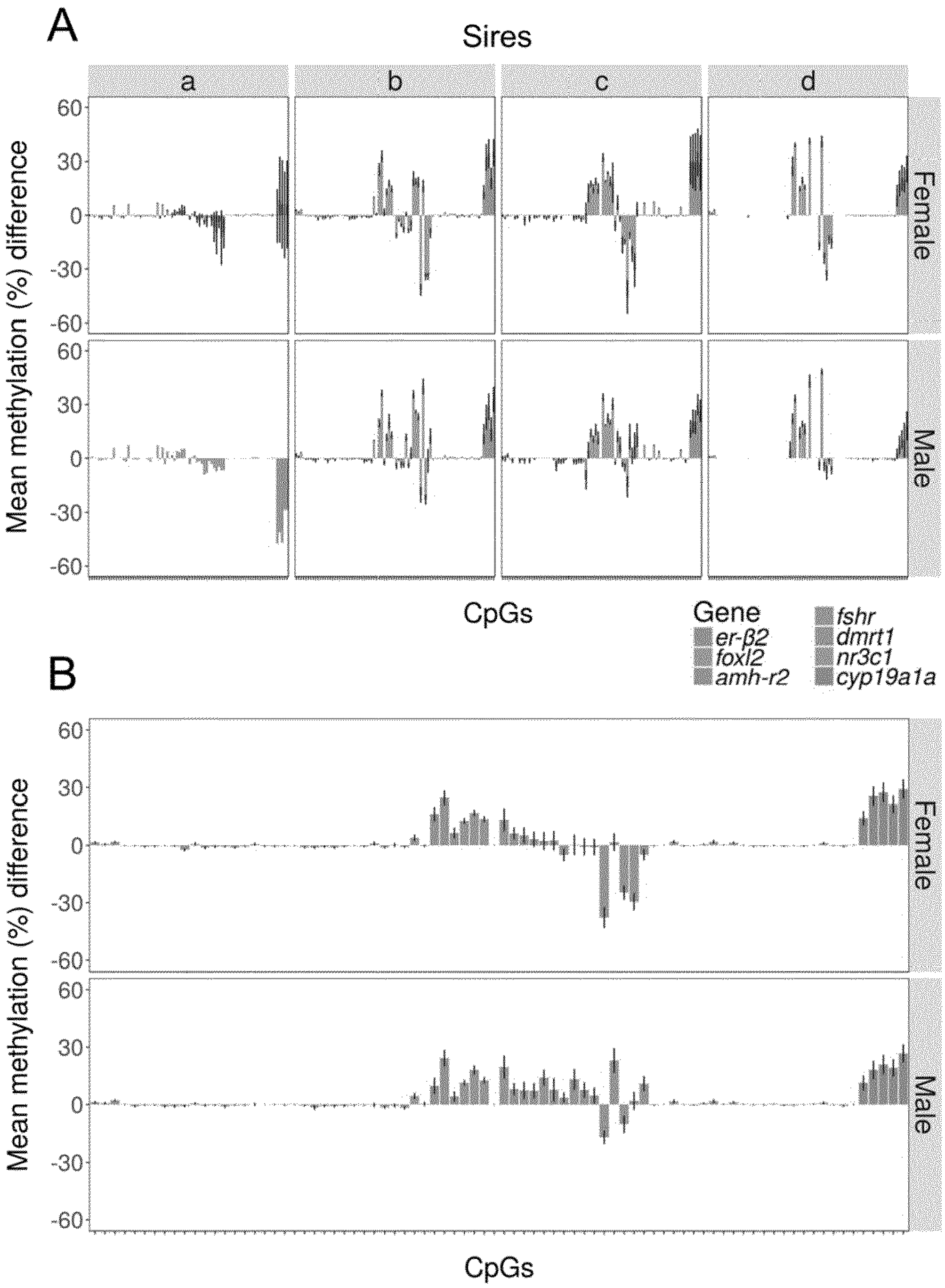
A
Sires
a
b
c
d
Mean methylation (%) difference
60
30
0
-30
-60
Female
Male
CpGs
Gene
er-β2
foxl2
amh-r2
fshr
dmrt1
nr3c1
cyp19a1a
B
Mean methylation (%) difference
Female
Male
CpGs

FIG. 7

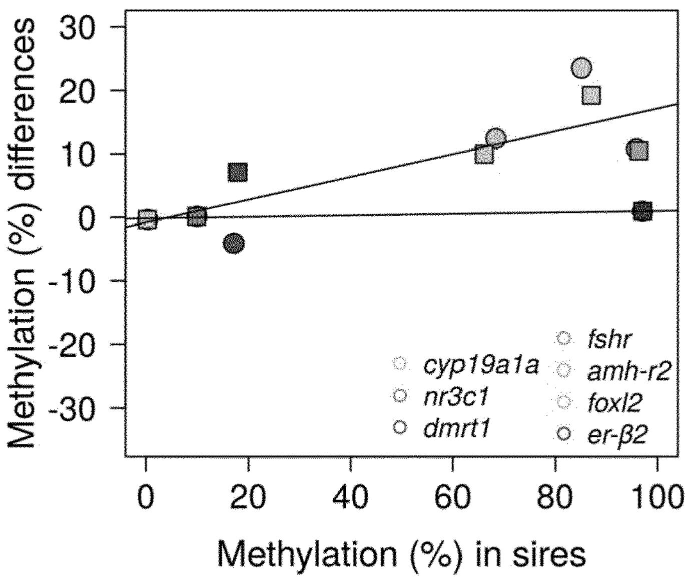
30
20
10
0
-10
-20
-30
Methylation (%) differences
0
20
40
60
80
100
Methylation (%) in sires
cyp19a1a
nr3c1
dmrt1
fshr
amh-r2
foxl2
er-β2

FIG. 8

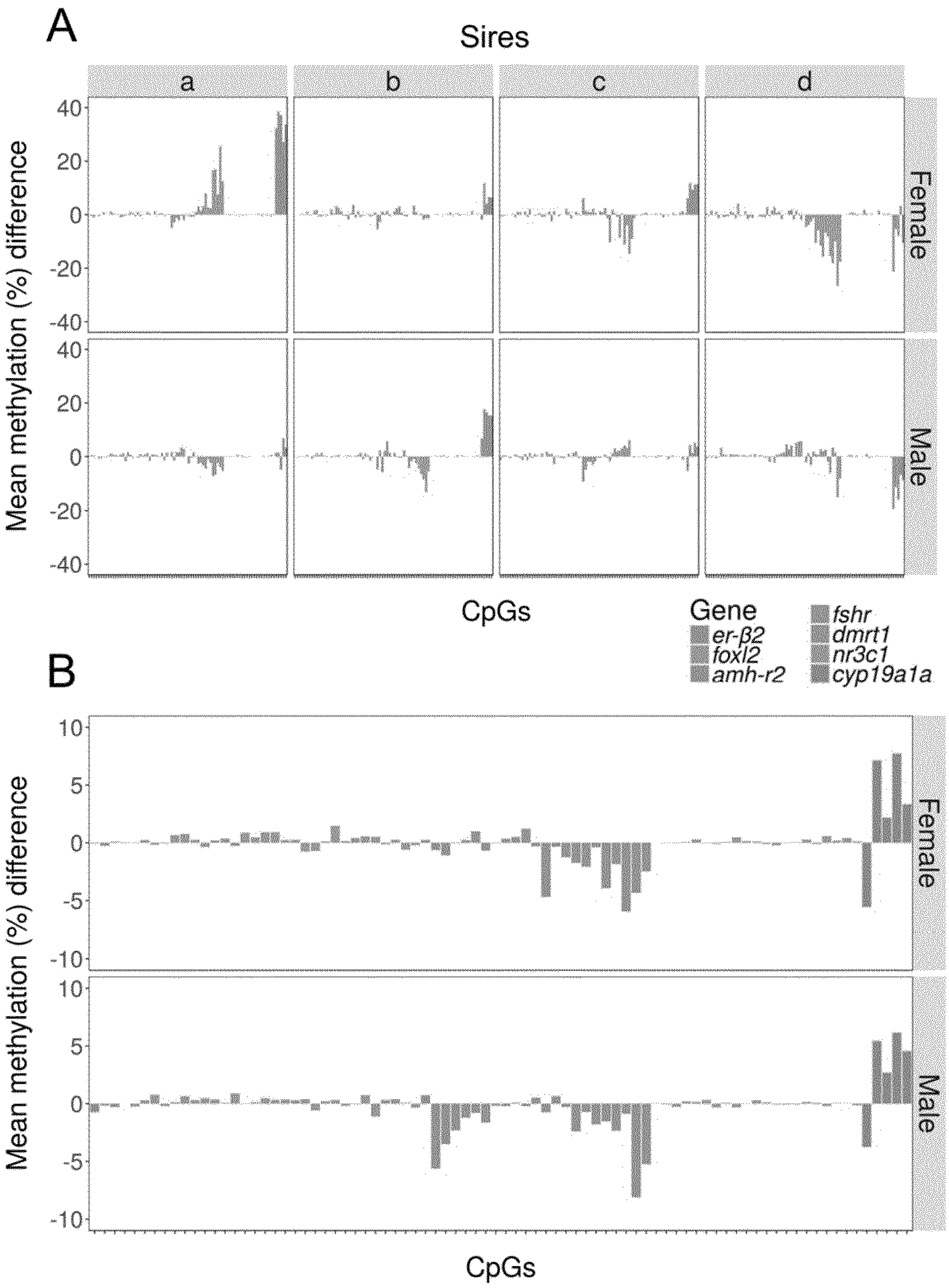
A
Sires
a
b
c
d
Female
Male
Mean methylation (%) difference
40
20
0
-20
-40
CpGs
Gene
er-β2
foxl2
amh-r2
fshr
dmrt1
nr3c1
cyp19a1a
B
10
5
0
-5
-10

FIG. 9

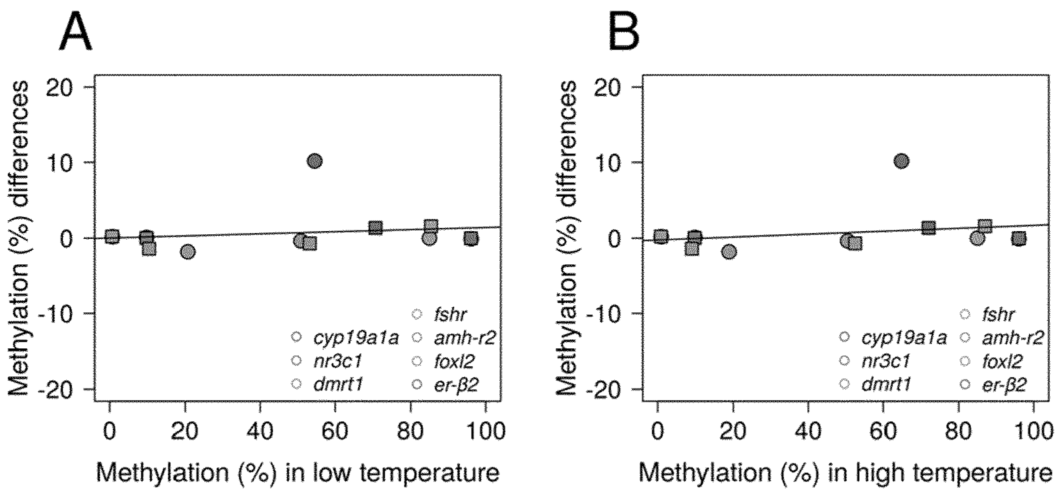
A
B
Methylation (%) differences
Methylation (%) in low temperature
Methylation (%) in high temperature
cyp19a1a
nr3c1
dmrt1
fshr
amh-r2
foxl2
er-β2

FIG. 10

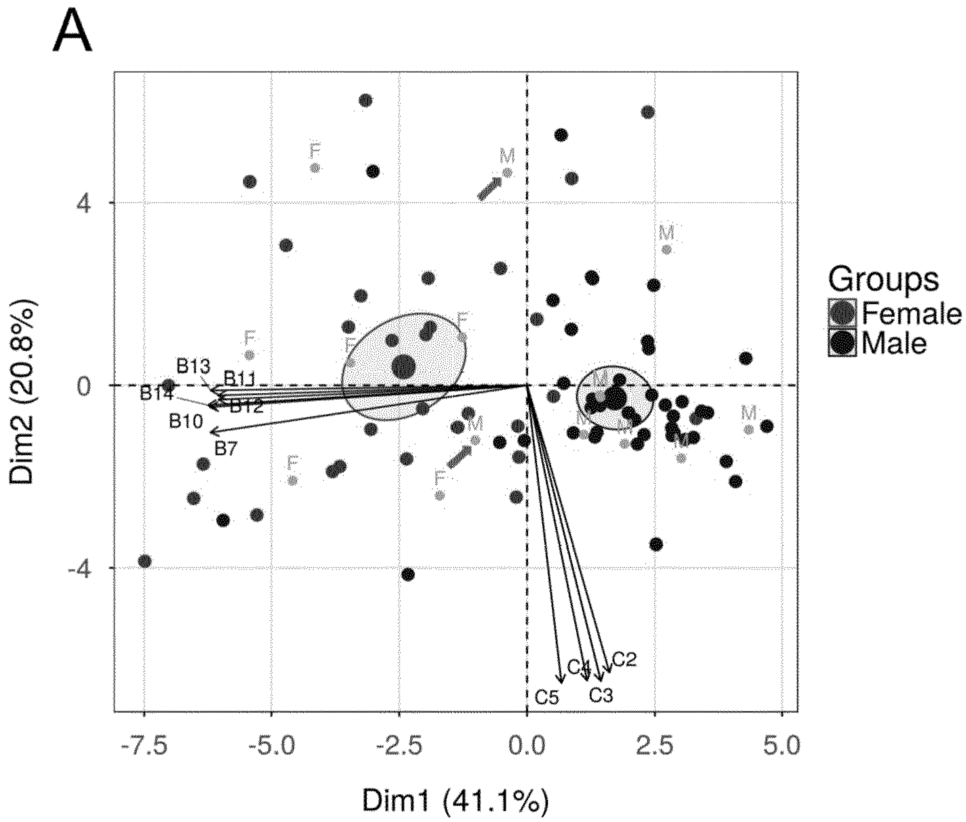
A
Dim2 (20.8%)
Dim1 (41.1%)
Groups
Female
Male
B13
B11
B14
B12
B10
B7
C2
C4
C3
C5

FIG. 11

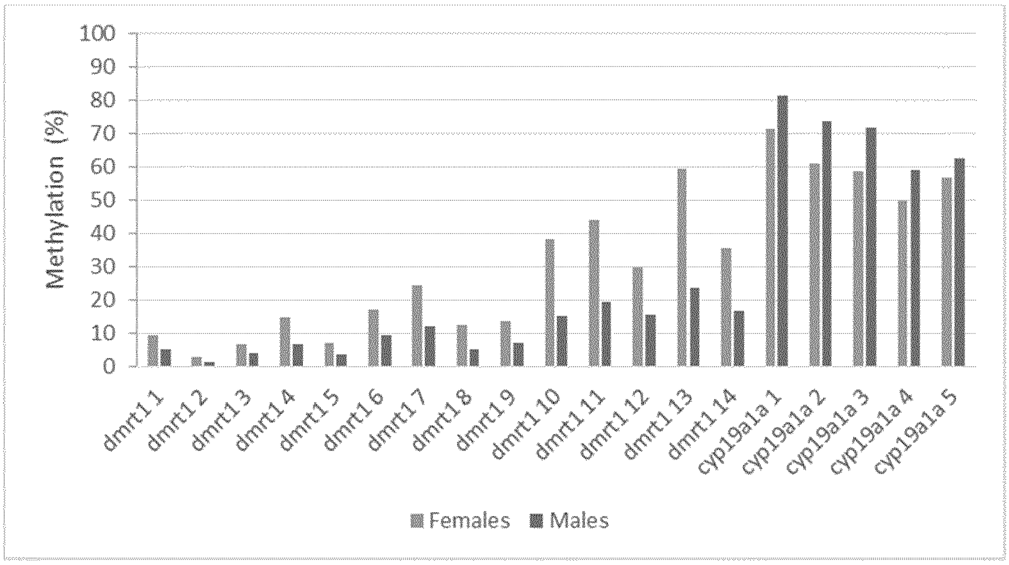
100
90
80
70
60
50
40
30
20
10
0
Methylation (%)
dmrt1 1
dmrt1 2
dmrt1 3
dmrt1 4
dmrt1 5
dmrt1 6
dmrt1 7
dmrt1 8
dmrt1 9
dmrt1 10
dmrt1 11
dmrt1 12
dmrt1 13
dmrt1 14
cyp19a1a 1
cyp19a1a 2
cyp19a1a 3
cyp19a1a 4
cyp19a1a 5
Females
Males

FIG. 12

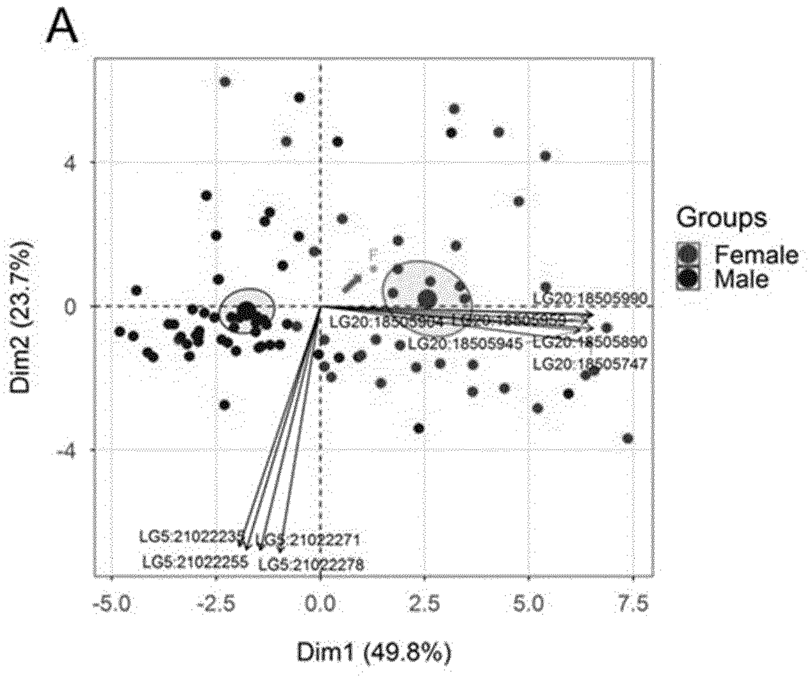
A
Dim2 (23.7%)
Dim1 (49.8%)
4
0
-4
-5.0
-2.5
0.0
2.5
5.0
7.5
Groups
Female
Male
LG20:18505990
LG20:18505904
LG20:18505945
LG20:18505890
LG20:18505747
LG5:21022235
LG5:21022255
LG5:21022271
LG5:21022278

FIG. 13

Europäisches Patentamt
European Patent Office
Office européen des brevets

# EUROPEAN SEARCH REPORT

Application Number
EP 18 38 2646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br>A | A. Y. WEN ET AL: "CpG methylation of dmrt1 and cyp19a promoters in relation to their sexual dimorphic expression in the Japanese flounder Paralichthys olivaceus : dmrt1 and cyp19a 's cpg methylation and expression",<br>JOURNAL OF FISH BIOLOGY.,<br>vol. 84, no. 1,<br>23 December 2013 (2013-12-23), pages 193-205, XP055547406,<br>NL<br>ISSN: 0022-1112, DOI: 10.1111/jfb.12277<br>* page 203, line 2 *<br>* page 197, paragraph 2 - page 200, paragraph 1; figures 1,2; table 1 *<br>----- | 11-14<br><br>1-10 | INV.<br>C12Q1/6876<br>C12Q1/6879 |
| Y<br><br>A | LAIA NAVARRO-MARTÍN ET AL: "DNA Methylation of the Gonadal Aromatase (cyp19a) Promoter Is Involved in Temperature-Dependent Sex Ratio Shifts in the European Sea Bass",<br>PLOS GENETICS,<br>vol. 7, no. 12,<br>29 December 2011 (2011-12-29), page e1002447, XP055547397,<br>DOI: 10.1371/journal.pgen.1002447<br>* page 2, column 2, paragraph 2 - page 3, column 2, paragraph 1; figures 1,3,8 *<br>* page 12, column 1, paragraph 3 *<br>----- | 11-14<br><br>1-10 | TECHNICAL FIELDS SEARCHED (IPC)<br>C12Q |

-/--

The present search report has been drawn up for all claims

2

| Place of search | Date of completion of the search | Examiner |
|---|---|---|
| Munich | 25 January 2019 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 38 2646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FEI-LONG WANG ET AL: "Genome-wide identification, evolution of DNA methyltransferases and their expression during gonadal development in Nile tilapia", COMPARATIVE BIOCHEMISTRY AND PHYSIOLOGY PART B: BIOCHEMISTRY AND MOLECULAR BIOLOGY, vol. 226, 28 August 2018 (2018-08-28), pages 73-84, XP055547339, AMSTERDAM, NL ISSN: 1096-4959, DOI: 10.1016/j.cbpb.2018.08.007 | 11-14 | |
| A | * page 80, column 2, paragraph 4.3 * ----- | 1-10 | |
| Y | XIAOWU CHEN ET AL: "Expression and DNA methylation analysis of cyp19a1a in Chinese sea perch Lateolabrax maculatus", COMPARATIVE BIOCHEMISTRY AND PHYSIOLOGY PART B: BIOCHEMISTRY AND MOLECULAR BIOLOGY, vol. 226, 9 August 2018 (2018-08-09), pages 85-90, XP055545690, AMSTERDAM, NL ISSN: 1096-4959, DOI: 10.1016/j.cbpb.2018.07.008 | 11-14 | TECHNICAL FIELDS SEARCHED (IPC) |
| A | * page 89, column 1, paragraph 2 - column 2, paragraph 1; figure 4 * ----- -/-- | 1-10 | |

The present search report has been drawn up for all claims

2

| Place of search | Date of completion of the search | Examiner |
|---|---|---|
| Munich | 25 January 2019 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 38 2646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG YI YA ET AL: "Epigenetic control ofcyp19a1aexpression is critical for high temperature induced Nile tilapia masculinization", JOURNAL OF THERMAL BIOLOGY, PERGAMON PRESS, OXFORD, GB, vol. 69, 21 June 2017 (2017-06-21), pages 76-84, XP085213363, ISSN: 0306-4565, DOI: 10.1016/J.JTHERBIO.2017.06.006 | 11-14 | |
| A | * page 77, column 2, paragraph 2.5; figures 2,3 * * page 80, column 1, paragraph 4.1 * ----- | 1-10 | |
| Y | C. SHAO ET AL: "Epigenetic modification and inheritance in sexual reversal of fish", GENOME RESEARCH, vol. 24, no. 4, 2 February 2014 (2014-02-02), pages 604-615, XP055546799, US ISSN: 1088-9051, DOI: 10.1101/gr.162172.113 | 11-14 | TECHNICAL FIELDS SEARCHED (IPC) |
| A | * page 605, column 2, paragraph 2; figures 2,3 * * the whole document * ----- | 1-10 | |
| | -/-- | | |

The present search report has been drawn up for all claims

2

| Place of search | Date of completion of the search | Examiner |
|---|---|---|
| Munich | 25 January 2019 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 4

Europäisches Patentamt
European Patent Office
Office européen des brevets

# EUROPEAN SEARCH REPORT

Application Number
EP 18 38 2646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | DAFNI ANASTASIADI ET AL: "Dynamic epimarks in sex-related genes predict gonad phenotype in the European sea bass, a fish with mixed genetic and environmental sex determination", EPIGENETICS, vol. 13, no. 9, 28 September 2018 (2018-09-28), pages 988-1011, XP055545681, US ISSN: 1559-2294, DOI: 10.1080/15592294.2018.1529504 * the whole document * ----- | 1-14 | |

TECHNICAL FIELDS SEARCHED (IPC)

The present search report has been drawn up for all claims

2

| Place of search | Date of completion of the search | Examiner |
|---|---|---|
| Munich | 25 January 2019 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 107217099

### Non-patent literature cited in the description


- **ALTSCHUL S.F. et al.** Basic local alignment search tool. *J Mol Biol.,* 05 October 1990, vol. 215 (3), 403-10
- **UNTERGASSER A et al.** *Nucleic Acids Res 2012,* 2012, vol. 40, e115
- **HOSOMICHI et al.** *BMC Genomics,* 2014, vol. 15, 645
- **TINE M et al.** *Nat Commun,* 2014, vol. 5, 5770
- **KRUEGER F ; ANDREWS SR.** Bismark: a flexible aligner and methylation caller for Bisulfite-Seq applications. *Bioinformatics,* 2011, vol. 27, 1571-2
- **HUBER W et al.** *Nat Methods,* 2015, vol. 12, 115-21
- **BUUREN S ; GROOTHUIS-OUDSHOORN K.** MICE: Multivariate Imputation by Chained Equations in R. *J Stat Softw,* 2011, https://www.jstatsoft.org/article/view/v045i03